# EUROPÄISCHE PATENTSCHRIFT

(11) **EP 2 167 964 B1**
(45) Veröffentlichungstag und Bekanntmachung des Hinweises auf die Patenterteilung: **11.03.2015**
(21) Anmeldenummer: 08761034.1
(22) Anmeldetag: 13.06.2008
(51) Int. Cl.: G01N 33/542, G01N 33/543

(54) **VERFAHREN ZUR DURCHFÜHRUNG UND AUSWERTUNG VON MIX&MEASURE ASSAYS FÜR DIE MESSUNG VON REAKTIONSKINETIKEN SOWIE VON KONZENTRATIONEN UND AFFINITÄTEN VON ANALYTEN IM MULTIPLEXFORMAT**
METHOD FOR CARRYING OUT AND EVALUATING MIX&MEASURE ASSAYS FOR THE MEASUREMENT OF REACTION KINETICS, CONCENTRATIONS AND AFFINITIES OF ANALYTES IN MULTIPLEX FORMAT
PROCÉDÉ POUR EXÉCUTION ET EXPLOITATION D'ESSAIS "MIX&MEASURE" POUR L'ÉVALUATION DES CINÉTIQUES DE RÉACTION AINSI QUE DES CONCENTRATIONS ET AFFINITÉS DES ANALYTES EN FORMAT MULTIPLEX

(30) Priorität: 13.06.2007 DE 102007027779; 29.06.2007 DE 102007031137; 08.02.2008 DE 102008008485
(43) Veröffentlichungstag der Anmeldung: 31.03.2010
(73) Patentinhaber: Attomol GmbH Molekulare Diagnostika, 03205 Bronkow (DE)
(72) Erfinder: LEHMANN, Werner, 03205 Bronkow (DE); BÖHM, Alexander, 01734 Rabenau (DE); GROSSMANN, Kai, 01157 Dresden (DE); HIEMANN, Rico, 01561 Thiendorf OT Sacka (DE); NITSCHKE, Jörg, 01998 Schipkau OT Klettwitz (DE); RÖDIGER, Stefan, 07546 Gera (DE)
(74) Vertreter: Gulde & Partner
(86) Internationale Anmeldenummer: PCT/EP2008/057515
(87) Internationale Veröffentlichungsnummer: WO 2008/152145

(56) Entgegenhaltungen:
- WO-A-00/16101
- WO-A-2004/027093

## Beschreibung

Die Erfindung betrifft allgemein das Gebiet der Nukleinsäureamplifikation, der Enzymologie und der Immunologie. Insbesondere betrifft die Erfindung ein Verfahren zur Echtzeitmessung (real time) von Reaktionskinetiken im Mix & Measure Testformat, wobei mehrere Parameter gleichzeitig (multiplex) aufgezeichnet und ausgewertet werden. Gegenstand der Erfindung sind auch ein Mehrfarbenfluoreszenzmesssystem mit thermischer Regelung, ein Beadarray sowie ein Kit zum Nachweis von Targetmolekülen im Array, vorzugsweise für Flüssigphasen-PCR, Festphasen-PCR und Multiplex-PCR.

Verfahren zur Bestimmung von Analyten in einer Probe werden in Echtzeit als Mix & Measure-Assays durchgeführt, um Kosten und Zeit bei der Durchführung der Bestimmung einzusparen oder die Genauigkeit sowie Robustheit der Teste zu verbessern. Derartige Tests werden z.B. für das pharmazeutische Hochdurchsatz-Screening oder die Nukleinsäureanalytik in der medizinischen Diagnostik eingesetzt.

Dabei ist es meist sehr wichtig, die Reaktionstemperatur zu regeln, d.h. die Temperatur konstant zu halten, einen Gradienten aufzubauen oder schnelle Thermozyklen identisch oder mit verschieden Verläufen zu temperieren während die Messparameter kontinuierlich aufgezeichnet und verarbeitet werden. Detektiert werden oft Fluoreszenzsignale, da diese in vielfältiger Weise mit dem Mix & Measure Assay-Format kombiniert werden können. Veränderungen von z.B. Fluoreszenzintensitäten, der Fluoreszenzlebensdauer oder der Fluoreszenzpolarisation können Indikatoren für z.B. sich verändernde Konzentrationen an Analyten in Proben oder für intermolekulare Wechselwirkungen sein und können kontaktfrei d.h. in unterschiedlichen Testformaten, im verschlossenen Reaktionsgefäß gemessen werden. Unter Mix & Measure Testformaten wird ein Analyseverfahren verstanden, bei dem alle reagierenden Komponenten bis zur Signalerfassung in einem Messkompartiment verbleiben.

Von Verfahren zur Erkennung und Mengenbestimmung von Nukleinsäuren, insbesondere die Polymerase-Kettenreaktion (PCR) ist bekannt, dass sie beispielgebend alle oben genannten Voraussetzungen benötigen, um optimal durchgeführt werden zu können. Die PCR ist sowohl für qualitative als auch für quantitative Aussagen einsetzbar. Eine Mengenbestimmung von PCR-Produkten kann auf unterschiedlichen Arten erfolgen. Neuere Detektionsmethoden erlauben es, die in vitro Synthese von PCR-Produkten "real time", d.h. homogen direkt im jeweils verwendeten PCR-Reaktionsgefäß in der Flüssigphase zu messen. Diese so genannte Realtime-PCR ist eine besonders sensitive Methode. Hierbei wird in jedem Zyklus der PCR die Entstehung von PCR-Produkten verfolgt. Die Messung der Amplifikation erfolgt dabei in der Regel in Thermozyklern, welche zusätzliche Mittel zur Messung von Fluoreszenzsignalen während der Amplifikationsreaktion aufweisen. Die Amplifikationsprodukte werden beispielsweise durch Fluoreszenz-markierte Hybridisationsproben, die lediglich bei Bindung an die Targetnukleinsäure fluoreszieren oder durch Doppelstrang-DNA bindende Fluoreszenzfarbstoffe detektiert. Ein definierter Signalschwellenwert wird für alle analysierten Reaktionen festgelegt und die zur Erreichung des Schwellenwerts notwendige Zykluszahl (Cp) sowohl für die Targetnukleinsäure als auch für die Referenznukleinsäuren bestimmt. Auf der Grundlage der für die Targetnukleinsäure sowie die Referenznukleinsäure erhaltenen Cp-Werte können so entweder absolute oder relative Kopienzahlen des Targetmoleküls bestimmt werden.

Ein neueres Verfahren zum Nachweis eines spezifischen Nukleinsäuremoleküls verwendet so genannte "Molecular Beacons" (Tyagi et al. US 6,150,097A) Molecular Beacons sind farbstoffmarkierte Oligonukleotide, die eine Stamm-Schleifen-Struktur haben. An den beiden freien Enden der Stammabschnitte (dem 3'- und dem 5'-Ende) ist jeweils ein Fluorophor gekoppelt, wobei der eine als Reporter-Farbstoff und der andere als Quencher-Farbstoff fungieren, der die Fluoreszenz des Reporter-Farbstoffs bei hinreichender räumlicher Nähe durch Förster Resonanz Energie Transfer (FRET) löscht. Die Sequenzen der Stammabschnitte an beiden Enden der Molecular Beacons sind so gewählt, dass dann, wenn sich der Molecular Beacon faltet, die Stammabschnitte ausschließlich aneinander, nicht aber mit anderen Abschnitten des Oligonukleotids hybridisieren. Der Abstand zwischen Reporter- und Quencher-Farbstoff ist im Zustand der hybridisierten Stammabschnitte hinreichend klein, so dass der Fluoreszenzfarbstoff auch bei geeigneter Anregung mit Licht nicht fluoresziert. Der Schleifenabschnitt weist eine Sequenz auf, die zur Sequenz einer Targetsequenz komplementär ist. Befinden sich die Molecular Beacons und die Targetsequenz aufweisenden Nukleinsäure-Moleküle in einer Lösung, können die Schleifenabschnitte und die Targetsequenz-Abschnitte hybridisieren, wodurch sich der Molecular Beacon unter Lösung der Hybridisierung der beiden Stammabschnitte entfaltet. Diese Entfaltung führt zur Vergrößerung des räumlichen Abstands zwischen dem Reporter-Farbstoff und dem Quencher-Farbstoff und der Reporter-Farbstoff wird zur Fluoreszenz angeregt. Bei kontinuierlicher Beobachtung der Fluoreszenzintensität kann ein Anstieg der Intensität festgestellt werden, wenn die Molecular Beacons die Targetsequenzen aufspüren und an diesen hybridisieren. So können die Nukleinsäure-moleküle quantitativ nachgewiesen werden. Diese Molecular Beacons haben den großen Nachteil einer aufwändigen und teuren Synthese, da diese Sonden sowohl mit dem Fluoreszenz- als auch mit dem Quencher-Farbstoff markiert werden müssen. Quencher-Fluoreszenzfarbstoff-Systeme werden in vergleichbaren Sondensystemen wie Amplifluor-Primer, Scorpions und TaqMan-Sonden genutzt.

Ein weiteres bekanntes System ist das Light-Cycler-System. Hier werden die Fluoreszenzfarbstoffe auf zwei verschiedene Oligonukleotide verteilt. Am 3'-Ende des ersten Oligonukleotides befindet sich der eine Fluoreszenzfarbstoff (z.B. Fluorescein), einen weiterer Fluoreszenzfarbstoff befindet sich am 5'-Ende des sich stromabwärts anlagernden Oligonukleotids (z.B. LC Red 640). Fluorescein wird von einer LED als Lichtquelle angeregt und emittiert Licht, welches über FRET das zweite Fluoreszenzmolekül anregt. Das von dem zweiten Farbstoff emittierte Licht wird über einen Fluoreszenzfilter mit Detektor gemessen. Eine Anregung des zweiten Farbstoffs kann nur dann erfolgen, wenn sich durch die Anlagerung der beiden Oligonukleotide an ihren Komplementärstrang die beiden Farbstoffmoleküle in räumlicher Nähe (Distanz 1 bis 5 Nukleotide) befinden. Das Prinzip dieses Systems beruht auf der sekundären Anregung eines zweiten Fluoreszenzfarbstoffs, die erst durch die Generierung des PCR-Produktes ermöglicht wird.

Die genannten Systeme sind grundsätzlich für Multiplex-Anwendungen einsetzbar, haben jedoch den Nachteil bei dem parallelen Nachweis verschiedener Zielsequenzen , dass der Reaktionsansatz wegen der steigenden Zahl der verschiedenen Detektionssonden relativ teuer wird und die zunehmende Zahl der Sonden die Amplifikationsreaktion beeinträchtigen kann. Außerdem sind nicht genügend FRET-Paare bekannt, um einen hohen Multiplex-Grad zu erzielen.

Bei der technischen Realisierung von Nukleinsäureassays für Routine-Untersuchungen sind von Bedeutung: der homogene Assay, das Multiplex-Verfahren sowie die Mikroarrays (auch Gen- oder Biochips genannt). In einer Multiplex-PCR mit dem Primer/Sondensystem bzw. dem Kit lassen sich mit dem oben beschriebenen Verfahren mehrere Primersätze kombinieren, die aufgrund unterschiedlicher Sequenzen die Detektion mehrerer Zielsequenzen, meist jedoch nicht mehr als fünf, in einem Reaktionsansatz erlauben. Qualitative Messungen erfolgen, indem nach einer vorher definierten Anzahl von Amplifikationszyklen geprüft wird, ob die Konzentration der aufgekoppelten Nukleinsäuremoleküle einen bestimmten Schwellenwert übersteigt. Für eine Quantifizierung wird diese Konzentration nach jedem Zyklus erfasst und die Anzahl der Zyklen bis zum Erreichen eines bestimmten Schwellenwertes bestimmt. Diese Anzahl ist ein Maß für die Konzentration der gesuchten Nukleinsäure in einer Probe.

Ihre weite Verbreitung verdankt die Realtime-PCR zwei Eigenschaften, die im Laboralltag nur selten gut zusammenpassen: sie ist schnell und präzise. Länger als zwei Stunden benötigt kaum ein Realtime-Cycler, um geringste DNA-Mengen zu vervielfältigen und zu quantifizieren. Realtime-Cycler arbeiten auch im Hochdurchsatz mit bis zu 384 PCR-Reaktionen gleichzeitig, deren Cyclerblöcke sich mit entsprechenden Mikrotiterplatten bestücken lassen.

Realtime-Cycler sind zahlreich bekannt. Sie ermöglichen eine Detektion von bis vorzugsweise zu fünf Fluoreszenzfarbstoffen gleichzeitig für Multiplex-Anwendungen, besitzen Heizblöcke vorzugsweise im Viererpack für 96er oder 384er-Well Mikrotiterplatten, zeigen schnelle Heiz-/Kühlraten die z.B. 30 PCR-Zyklen in 30 Minuten möglich machen, Quantifizierungssoftware, Schmelzpunktanalyse und vieles mehr. Das erfindungsgemäße System und Verfahren ist auch für Hochdurchsatzlabore geeignet, die sehr viele Proben abarbeiten müssen, wodurch bis zu 5000 Analysen pro Tag mit entsprechenden automatisierten Realtime-PCR-Robotern möglich sind. Wird die Realtime-Quantifizierung nicht benötigt und besteht nur Interesse an der Endpunkt-PCR , dann können bis zu 30.000 Proben in knapp 3 Stunden durch entsprechende PCR-Roboter ermöglicht werden.

Eine Alternative den Multiplex-Grad zu erhöhen ist die Hybridisierung der PCR-Produkte an einen DNA-Array, der auf seiner Oberfläche an räumlich distinkten Punkten spezifische Fangsonden trägt. Durch den Nachweis einer Hybridisierungsreaktion lassen sich amplifizierte DNA-Moleküle nachweisen oder in ihrer Sequenz charakterisieren.

Das Prinzip eines Array-Experiments besteht darin, alle auf dem Array befindlichen Genproben simultan mit einer Nukleinsäureprobe zu hybridisieren. Entscheidend ist hierbei, dass die durch reverse Transkription von RNA aus Zellen oder Gewebe gewonnene cDNA im Idealfall alle dort spezifisch exprimierten Gene umfasst. Das parallele Hybridisieren einer Nukleinsäureprobe mit einer Vielzahl von komplementären Genproben auf einem DNA-Array führt zu einem charakteristischen Hybridisierungsmuster mit entsprechender Hybridisierungsintensität. Hier zeigt sich der entscheidende Vorteil dieser Technologie gegenüber anderen Methoden zur Untersuchung von Genexpression. Während sich herkömmliche Methoden auf die Untersuchung einzelner Gene beschränken, liefert ein DNA-Array ein umfassenderes Genexpressionsprofil der untersuchten Zelle bzw. des untersuchten Gewebes. Besonders interessant ist die hierdurch gegebene Möglichkeit, die Interaktion verschiedener Gene zu untersuchen. Das Erfassen von Unterschieden in der Genexpression ist durch das gleichzeitige Hybridisieren von normalen/immortalisierten oder foetalen/adulten Zellen möglich.

Das Grundelement derzeit verwendeter Chips besteht vorzugsweise aus einem Glas- oder Kunststoffträger, wie sie in der Mikroskopie verwendet werden. Auf einen solchen Träger werden die Sonden (bevorzugt an Mikropartikeln) z.B. von einem Roboter oder lithografisch aufgebracht. Die Verfahren müssen hoch präzise und sauber arbeiten. Für jeden Spot werden Milliardstel-Liter (normalerweise 0,6 nl) der Oligonukleotid-Lösung verwendet. Nach jedem Durchgang findet eine Selbstreinigung der Nadeln (Spülung, Ultraschall) statt, um keine Verunreinigungen zu verschleppen. An einem Ende der Oligonukleotide befindet sich eine reaktive Gruppe wie z.B. eine Aminogruppe (NH₂). Diese verbindet sich mit einer chemischen Reaktion dauerhaft auf dem Träger bzw. an dem Mikropartikel. Diese Spots haben einen Durchmesser von bevorzugt 100 µm (0,1mm). Aufgrund dieser geringen Menge und des kleinen Abstandes zwischen den einzelnen Punkten (vzw. 0,3mm) haben auf einem Glasträger bis zu 30.000 Spots Platz. Diese dienen als spätere Andockstellen für die Proben-DNA. Die unbenutzte Fläche des Trägers muss abschließend blockiert werden. Das geschieht durch eine Behandlung mit einer speziellen Blockierungslösung, die bereits im fertigen Kit enthalten sein kann. Mit Hilfe der PCR werden die spezifischen Targetgene vermehrt, mit einer Fluoreszenz gekoppelt, auf die Träger aufgetragen und zur Hybridisierung inkubiert. Gegebenenfalls wird durch mehrere Waschungen ungebundene DNA entfernt. Ein spezieller Scanner bestrahlt die Chips mit Licht einer passenden Wellenlänge, die Fluoreszenzfarbstoffe werden angeregt und fluoreszieren. Das daraus resultierende Ergebnis wird anschließend ausgewertet, leuchten die entsprechenden Spots ist das Ergebnis positiv.

Neben ortskodierten Arrays, bei denen die Biomoleküle in feststehender Anordnung auf planare Träger z.B. aufgedruckt werden, wird beim Beadarray ein kodierter mit Partikeln bestückter Träger eingesetzt. Partikel mit verschiedenen Kodierungen werden für den Multiplex-Ansatz mit unterschiedlichen Biomolekülen beschichtet. Die Partikel selbst werden danach zufällig verteilt auf einen planaren Träger immobilisiert. Nach der Bindung von Molekülen aus der zu analysierenden Probe an die jeweiligen Partikel und deren Markierung (Ligandenmarkierung) erfolgen die Detektion der Kodierung, der Liganden und darüber die Zuordnung der Ligandenbindung zu den immobilisierten Biomolekülen.

Die Auswertung der Arrays ist ein vergleichsweise zeitaufwendiger Prozess. Insbesondere bei kinetischen Messungen mit mehreren Messpunkten in einer Messung entstehen inakzeptable Zeitverzögerungen, weil Bilder sehr aufwändig aufgenommen und verarbeitet werden. Da solche Testsysteme meist miniaturisiert werden, um Materialkosten, die durch das Multiplexing entstehen zu reduzieren, ist eine ausreichend hohe optische Auflösung erforderlich, was große Datenmengen verursacht. Ein weiterer Nachteil der Arrays ist deren mangelnde Reproduzierbarkeit in der Herstellung und die damit verbundene Schwankung der Messwerte, die durch den Einsatz von Arrays erhoben werden. Hierbei besitzen Beadarrays im Vergleich zu den planaren Assays Vorteile, die Auswertung ist durch die Kodierung der partikulären Träger jedoch langsamer, da mehrere Bilder (meist 3-4) pro Objekt aufgezeichnet und verarbeitet werden müssen. Es kommt hinzu, dass Arrays bislang noch selten als Mix & Measure Assays betrieben werden können, was Handlingaufwand und im Falle von Nukleinsäure-Arrays Kontaminationsgefahr bedeutet. Die Quantifizierung der Resultate ist deshalb auch nur sehr schwer möglich.

Der Erfindung lag deshalb die Aufgabe zugrunde, ein verbessertes Assay-System für Multiplex-Analysen bereitzustellen, welches mindestens eine der folgenden Anforderungen erfüllt:
- robuste Array-Plattform
- Mix & Measure Detektionsverfahren für Biomoleküle
- Multiplexfähiges, schnell temperierbares Messsystem
- auf die Hardware des Messsystems abgestimmte ultraschnelle Datenanalyse die eine Reduktion der Daten pro Objekt und Messpunkt zulässt sowie den Umfang der Bildverarbeitung auf ein Minimum beschränkt.

Die Erfindung dient bevorzugt zur Lösung analytischer Fragestellungen, die eine Aufzeichnung mehrerer Messpunkte pro Messreihe erfordern, wie z.B. bei kinetischen Messungen oder thermozyklischen Verfahren, oder wenn ein hoher Probendurchsatz wie z.B. beim Screening für das pharmazeutische Wirkstoffscreening gewährleistet werden muss. Die Erfindung soll dazu beitragen, dass nicht der Messvorgang der geschwindigkeitsbestimmende Schritt ist, sondern die Analyse abhängig von der Fragestellung an das Testprinzip optimal angepasst werden kann. Gelingt dies nicht, könnten schnelle Kinetiken nicht im Multiplex-Format bearbeitet werden oder die Reaktionen selbst würden zu unspezifischen Produkten führen, selbst wenn die Reaktion durch eine thermozyklische Steuerung quasi für die Messung unterbrechen wird.

Die Erfindung löst diese Anforderungen durch die Bereitstellung eines Verfahrens gemäß den Ansprüchen 1 bis 14.

Die Aufgabe wird erfindungsgemäß gelöst durch Bereitstellung eines Verfahrens zur Multiplex-Analyse von mehreren Analyten.

Erfindungsgemäß umfasst ein solches Verfahren folgende Schritte:
a. Einsatz eines Trägers, auf dem mindestens zwei verschiedene Mikropartikelpopulationen immobilisiert sind, wobei sich die verschiedenen Mikropartikelpopulationen in ihrer Fluoreszenzkodierung unterscheiden und mindestens zwei der unterschiedlich fluoreszenzkodierten Mikropartikelpopulationen jeweils Mikropartikel enthalten, die mit einer bestimmten, spezifischen Akzeptormolekülpopulation besetzt sind, wobei sich die Akzeptormolekülpopulationen der mindestens zwei unterschiedlichen fluoreszenzkodierten Mikropartikelpopulationen voneinander unterscheiden.
b. Messung der Fluoreszenz des Trägers aus Schritt a) mit einer optischen Auflösung 1 vor Inkontaktbringen des Trägers mit der zu analysierenden Probe,
   wobei die Auflösung 1:
   - eine Unterscheidung vom Mikropartikel-Singletten, -Dubletten,-Tripletten, -Multipletten und -Monolayern erlaubt, und
   - die Bestimmung der örtlichen Lage der einzelnen immobilisierten Mikropartikel der jeweiligen mindestens zwei unterschiedlichen Mikropartikelpopulationen auf dem Träger unter Berücksichtigung der jeweils unterschiedlichen Fluoreszenzkodierung der mindestens zwei verschiedenen Mikropartikelpopulationen gestattet.
c. Inkontaktbringen des Trägers aus Schritt a) mit der zu analysierenden Probe, wobei die Interaktion des jeweiligen Analyten mit dem für ihn spezifischen Akzeptormolekül auf dem entsprechenden immobilisierten Mikropartikel eine Fluoreszenzänderung hervorruft, ggf. kann Schritt c) auch vor Schritt b) erfolgen.
d. Vornahme mindestens einer weiteren Messung der Fluoreszenz des Trägers während oder nach dem Inkontaktbringen gemäß Schritt c) mit einer Auflösung 2.
e. Zuordnung der mit Auflösung 2 gemessenen Fluoreszenzwerte zu den gemäß Schritt b) örtlich auf den Träger identifizierten und einer bestimmten Akzeptormolekülpopulation zugeordneten, einzelnen Mikropartikel-Singletten, - Dubletten, -Tripletten, -Multipletten und -Monolayern.
f. Bestimmung der Fluoreszenzänderung durch Inkontaktbringen gemäß Schritt c) für jede örtlich identifizerte Mikropartikel-Singlette, -Dublette, -Triplette, - Multiplette und -Monolayer auf dem Träger.

Das Verfahren beruht darauf, dass Mikropartikelpopulationen dadurch schnell unterscheidbar gemacht werden, dass diese Mikropartikel bei Anregung mit geeignetem Licht ein populationsspezifisches Fluoreszenzmuster abgeben. Mittels einer fluoreszenzoptischen Messung kann dann jeder Mikropartikel auf einer geeigneten Oberfläche schnell in seiner örtlichen Lage und Anordnung eindeutig bestimmt werden und einer bestimmten Mikropartikelpopulation zugeordnet werden. Es werden so viele voneinander unterscheidbare Mikropartikelpopulationen im erfindungsgemäßen Verfahren eingesetzt, wie Analyte und Kontrollen in der zu analysierenden Probe detektiert werden sollen. Jede unterscheidbare Mikropartikelpopulation wird mit Molekülen einer bestimmten spezifischen Akzeptormolekülpopulation besetzt, wobei jede spezifische Akzeptormolekülpopulation mit einem zu bestimmenden Analyt spezifisch interagiert oder als Kontrolle dient. Jede unterscheidbar fluoreszenzkodierte Mikropartikelpopulation ist nun jeweils mit genau einer spezifischen Akzeptormolekülpopulation besetzt. Die unterschiedlichen, jeweils mit Akzeptormolekülen einer bestimmten Spezifität besetzten Mikropartikelpopulationen werden dann auf einem geeigneten Träger immobilisiert, ohne dass eine gezielte räumliche Anordnung der unterschiedlichen Mikropartikelpopulationen auf dem Träger notwendig ist. Nach erfolgter Immobilisierung, wird eine erste Messung der Fluoreszenz des bestückten Trägers durchgeführt. Diese Messung dient als Referenz für spätere Messungen im Verfahren und erlaubt anhand der eindeutigen Fluoreszenzkodierung der einzelnen Mikropartikelpopulationen nun die genaue räumliche Bestimmung der Lage jedes einzelnen immobilisierten Mikropartikels, dessen Anordnung z.B. als Mikropartikel-Singlette, -Dublette, -Triplette, -Multiplette oder als -Monolayer und die exakte Zuordnung des betreffenden Partikels zu einer bestimmten, mit einer spezifischen Akzeptormolekülpopulation besetzten Mikropartikelpopulation. Anschließend kann der Träger mit der zu analysierenden Probe in Kontakt gebracht werden. Durch die spezifische Interaktion des Analyten mit seinem Akzeptormolekül auf der Oberfläche des betreffenden Mikropartikels oder mit einem bestimmten Liganden für dieses Akzeptormolekül kommt es zu einer Fluoreszenzänderung. Diese Fluoreszenzänderung wird gemessen in einer oder einer Reihe von zeitlich aufeinanderfolgenden Messungen. Durch einen ortsgenauen Vergleich der Referenzmessung und der späteren Probenmessung(en) kann die Fluoreszenzänderung für jeden einzelnen Mikropartikel bestimmt und einer bestimmten Mikropartikelpopulation eindeutig zugeordnet werden.

Dieses Verfahren kann z.B. zur Konzentrations- bzw. Affinitätsbestimmung von Analyten in einer Probe oder zur Bestimmung von Reaktionskinetiken verwendet werden.

Die einzelnen Schritte und Bestandteile des erfindungsgemäßen Verfahrens werden im folgenden näher beschrieben.

### Multiplex-Analyse

Der bevorzugt realisierte Multiplex-Grad der Analysen unter Verwendung des erfindungsgemäßen Verfahrens beträgt 2-1000 unterschiedliche Analyte und Kontrollen pro Probe, wobei mehrere Messparameter wie z.B. verschiedene Epitope oder Nukleotidsequenzbereiche eines Analyten gleichzeitig erfasst werden können. Der bevorzugte Multiplex-Grad beträgt 2-100 unterschiedliche Analyten und Kontrollen pro Probe und besonders bevorzugt 2-10 Analyte und Kontrollen pro Probe.

### Analyte

Analyte können alle denkbaren organischen und anorganischen Moleküle einer Probe sein, die über ihre spezifische Interaktion mit den Akzeptormolekülen auf der Oberfläche der immobilisierten Mikropartikel und der dadurch verursachten Fluoreszenzänderung detektiert werden können.

Bevorzugte Analyte sind Peptide, Proteine, Enzyme, Lektine, Antikörper, Antigene, Aptamere, Polysaccharide, Lipide oder Nukleinsäuren . Im Fall von Nukleinsäuren sind bevorzugt natürliche und synthetische DNA- oder RNA-Moleküle, besonders bevorzugt sind Nukleinsäuren, die bei Amplifikationsreaktionen, wie z.B. PCR-Reaktionen in jeden Zyklus entstehen. Es können aber auch nicht natürliche Nukleinsäureanaloga oder Aminosäureanaloga sowie Moleküle die diese enthalten zum Einsatz kommen.

Dabei können z.B. entweder die zu analysierenden Analyte selbst markiert sein und durch ihre Markierung und ihre spezifische Interaktion mit dem Akzeptormolekül direkt eine Fluoreszenzänderung hervorrufen. Oder markierte oder unmarkierte Analyte rufen eine Fluoreszenzänderung dadurch indirekt hervor, dass die Analyte, z.B. durch ihre spezifische Interaktion mit den Akzeptormolekülen oder durch eine spezifische Interaktion mit einem konkurrierenden Liganden für das spezifische Akzeptormolekül, die Bindung eines entsprechend markierten Liganden beeinflussen oder verhindern.

### Mix-&-Measure-Testformat

Die Testdurchführung vereinfacht sich dadurch, dass das erfindungsgemäße Verfahren bevorzugt als Mix & Measure-Verfahren abläuft. Das heißt, alle Reaktanden werden in das Reaktionsgefäß gegeben und abgesehen vom gegebenenfalls notwendigen Verschließen des Reaktionsgefäßes und der Messung und Auswertung sind keine weiteren Arbeitsschritte notwendig.

### Träger

Die Reaktionen können auf planaren Trägern oder in Vertiefungen dieser Träger ablaufen und detektiert werden. Die Träger können als Objektträger, Blister, Mikrotestplatte, Kapillare oder Tube strukturiert sein. Der Verschluss der Vertiefungen kann durch Deckel, aufgeschweißte Folien oder auch durch Überschichtung erfolgen. Besonders bevorzugt werden NucleoLink-Platten (Nunc) für die Nukleinsäureamplifikation eingesetzt, die eine Oberflächen-Beschichtung für die kovalente Immobilisierung von Biomolekülen tragen einen ausreichend planaren und transparenten Gefäßboden besitzen und thermostabil sind. Oberflächenbeschichtungen können auch eingesetzt werden, um die unspezifische Bindung von Probenmolekülen oder Detektionsreagenzien zu verringern. Eine Mikrostrukturierung der Oberfläche, die die optische Fokussierung nicht beeinträchtigt, ist natürlich möglich.

Der feste Träger kann in irgendeiner Form vorliegen, und kann aus verschiedenen Materialien bzw. Werkstoffen bestehen, die insbesondere verschiedene Metalle, Glas und Kunststoffe umfassen. Bevorzugte feste Träger sind Nylonmembranen, Epoxidglas und Borfluoratglas. Der Vorteil der Verwendung von Glas und Kunststoffen kann in der Durchsichtigkeit der Materialien liegen, die die Herstellung von Trägern in der Art von Objektträgern oder Mikroplatten für den parallelen Hochdurchsatz von Proben und einer daraus herrührenden Kostenverringerung ermöglicht. Die Mikroarrays können in Form eines Objektträgers oder einer Mikroplatte (ebenfalls als Mikrotiterplatte bezeichnet) vorliegen. Bei der Mikroplatte handelt es sich um einen geschüsselten (dished) Behälter mit mehreren (mindestens zwei) Vertiefungen. Bei auf Mikroplatten basierenden Mikroarrays handelt es sich um eine Mikroplatte mit mehreren Vertiefungen, in deren Böden in den Mikroarray-Biochip platziert ist. Ein Beispiel der Mikroplatte ist eine gut bekannte ELISA Mikrotiterplatte mit 96 Vertiefungen.

Weiterhin sind auf selbst-anordnenden Schichtsystemen basierende feste Träger ebenso für die Implementierung der vorliegenden Erfindung geeignet. Die Anwendung kann unter Verwendung automatischer Verfahren erfolgen.

Die Träger können aus Polycarbonat, Polystyrol, (weitere Kunststoffe), Glas. Metall, Keramik bestehen.

### Fluoreszenzkodierte Mikropartikel

Die Verfügbarkeit von reaktiven oder nichtreaktiven Additiven flüssiger Kunststoffe, z. B. Thermoplaste, Elastomere, Duromere, lässt sich effizient durch Umhüllung oder Einbettung in linearkettige oder netzwerkbildende Polymere steuern. Derartige polymerbasierte Mikrokomposite sind in Form von Mikrokapseln mit Kern-Schale-Struktur bzw. von mikroskaligen Matrixpartikeln mit weitgehend homogener Verteilung der Komponenten über den Partikelquerschnitt bekannt (Ch.A.Finch, R.Bodmeier: "Microencapsulation" in Ullmann's Encyclopedia of Industrial Chemistry, 6. Ed. 2001 Electronic Release). Der Kern von Mikrokapseln kann in fester, flüssiger oder gasförmiger Form (Hohlkugeln) vorliegen. Bei Matrixpartikeln sind homogen- und heterogenphasige Systeme bekannt. Erfindungsgemäß kommen alle Mikropartikel in Frage, die eine Immobilisierung ermöglichen. Die Partikelteilchen bestehen vorzugsweise aus einem Polymermaterial. Bevorzugt sind folgende Polymermaterialien, welche umfassen, jedoch nicht hierauf beschränkt sind: Polystyrol, Polyacrylsäure, Polyacrylnitril, Polyamid, Polyacrylamid, Polyacrolein, Polybutadien, Polycaprolacton, Polycarbonat, Polyester, Polyethylen, Polyethylenterephthalat, Polydimethylsiloxan, Polyisopren, Polyurethan, Polyvinylacetat, Polyvinylchlorid, Palyvinylpyridin, Polyvinylbenzylchlorid, Polyvinyltoluol, Polyvinylidenchlorid, Polydivinylbenzol, Polymethylmethacrylat, Polylactid, Polyglycolid, Poly(lactid-co-glycolid], Polyanhydrid, Polyorthoester, Polysulfon oder Kombinationen derselben. Andere Polymermaterialien, wie zum Beispiel Kohlehydrate, wie Carboxymethylcellulose, Hydroxyethylcellulose, Agar, Gel, ein eiweißartiges Polymer, Polypeptide, eukaryotische und prokaryotische Zellen, Lipide, Metall, Harze, Latex, Kautschuk, Silikon, wie zum Beispiel Polydimethyldiphenylsiloxan, Glas, Melamin, Keramik, Holzkohle, Kaolinit, Bentonit und dergl. können in gleicher Weise verwendet werden. In diese Polymeren können auch ein Magnet oder ein aus der Gruppe ausgewähltes magnetisch ansprechbares Metalloxid eingearbeitet werden, das aus superparamagnetischem, paramagnetischem oder ferromagnetischem Metalloxid besteht. Die Partikel können zusätzliche funktionelle Gruppen an der Oberfläche enthalten, wie zum Beispiel Carboxylate, Ester, Alkohole, Carbamide, Aldehyde, Amine, Schwefeloxide, Mercaptogruppen, Stickoxide oder Halogenide, die eine Bindung analytischer Reaktanden und/oder ein Binden von Teilchen an Teilchen erleichtern können.

Verfahren zur Herstellung von Mikropartikeln mittels reaktiver und nichtreaktiver Partikelbildungsprozesse sind vielfach beschrieben. Bei der reaktiven Partikelbildung erfolgt die Bildung der Wand oder der Matrix parallel zu einem Polymerisations-, Polykondensations- oder Polyadditionsprozess. Bei den nichtreaktiven Verfahren werden filmbildende Polymere direkt eingesetzt, die auf thermodynamische Weise zur Phasenseparation und zur Partikelbildung gebracht werden. In Verfahren zur Verkapselung fester oder flüssiger Kernmaterialien werden z.B. Melamin-Formaldehyd-Harze eingesetzt. Insbesondere Melamin-Formaldehyd-Harze sind vielfältig und problemlos einsetzbar, und sie können zur Partikelbildung aus wässriger Phase appliziert werden. Die Mikrokapselgröße kann in Abhängigkeit von den Reaktionsbedingungen, z.B. Emulgatorzusatz oder Dispergiermethode eingestellt werden und liegt für die erfindungsgemäße Verwendung zwischen 0,5 und 30 µm. Eine weitere Möglichkeit ist die hydrothermale Vernetzung von Hydroxymethyl-Melamin.

Fluoreszenzkodierte Mikropartikel sind dem Fachmann bekannt und z.B. in DE 699 07 630 T2 und DE 10054382.0 beschrieben. Die Farbstoffe, wenn mehr als ein Farbstoff zum Färben von mehr als einer Population von Partikeln verwendet wird, sind derart ausgewählt, dass sie wesentlich verschiedene Emissionsspektren besitzen, vorzugsweise mit Emissionsmaxima, welche um mehr als 10 nm, bevorzugter mit Emissionsmaxima, die um mehr als 25 nm, getrennt sind, noch bevorzugter um mehr als 50 nm getrennt sind. Die Farbstoffe können ausgewählt werden, um Emissionsbanden aufzuweisen, welche zu im Handel erhältlichen Filtern passen, oder zum Nachweis von Mehrfachfluorophoren mit verschiedenen Erregungs- und Emissionsbanden.

Hier eine Liste von Fluoreszenzfarbstoffklassen einfügen.

Fluoreszenzfarbstoffe, die der Markierung der Mikropartikel dienen, sind alle Substanzen, die detektierbare Lumineszenzsignale senden können. Es können jedoch auch Farbstoffe eingesetzt werden, die Röntgenstrahlung emittieren oder eine Phosphoreszenz aufweisen. Fluoreszenzfarbstoffe im Sinne der Erfindung sind alle gasförmigen, flüssigen oder festen anorganischen und/oder organischen Verbindungen, die dadurch gekennzeichnet sind, daß sie nach Anregung die absorbierte Energie in Form von Strahlung von gleicher, längerer oder kürzerer Wellenlänge wieder abgeben. Das heißt, auch anorganische oder organische lumineszenzfähige Pigmente oder "Quantum dots" können als Fluoreszenzfarbstoffe im Sinne der Erfindung verwendet werden. Es kann jedoch auch vorgesehen sein, daß die Mikropartikel so beschaffen sind, daß sie eine Eigenfluoreszenz, bzw. sowohl eine Eigenfluoreszenz als auch eine nichteigene Fluoreszenzmarkierung aufweisen. Eine Eigenfluoreszenz der Mikropartikel kann beispielsweise dadurch erzeugt werden, daß die Mikropartikel das Mineral Fluorit beinhalten. Als nichteigene Fluoreszenzfarbstoffe können beispielsweise eingesetzt werden: Dansylchlorid, Fluoresceinisothiocyanat, 7-Chlor-4-nitrobenzoxadiazol, Pyrenbutyrylessigsäure-anhydrid, N-Iodoacetyl-N'-(5-sulfonsäure-1-naphthyl)-ethylendiamin, 1-Anilinonaphthalen-8-sulfonat, 2-Toluidinonaphthalen-6-sulfonat, 7-(p-Methoxybenzylamino)4-nitro-benz-2-oxa-1,3-diazol, Formycin, 2-Aminopurinribonukleosid, Ethenoadenosin, Benzoadenosin, α- und β-Parinarsäure und/oder Δ9,11,13,15Octaecatetraensäure, Cadmiumselenit-Kristalle einer oder unterschiedlicher Größen und andere. Als Fluoreszenzfarbstoffe können ebenfalls z.B. Übergangsmetallkomplexe eingesetzt werden, die folgende Substanzen enthalten: Ruthenium (II), Rhenium (I) oder Osmium und Iridium als Zentralatom und Diiminliganden; phosphoreszierende Prophyrine mit Platin, Palladium, Lutetium oder Zinn als Zentralatom; phosphoreszierende Komplexe der Seltenerden wie Europium, Dysprosium oder Terbium; phosphoreszierende Kristalle wie Rubin, Cr-YAG, Alexandrit oder phosphoreszierende Mischoxide wie Magnesiumfluorogermanat bzw. Cadmiumselenit-Kristalle, Fluorescein, Aminofluorescein, Aminomethylcoumarin, Rhodamin, Rhodamin 6G, Rhodamin B, Tetramethylrhodamin, Ethidiumbromid und/oder Acridinorange. Als Fluoreszenzfarbstoffe können z.B. folgende Stoffe in Kombination eingesetzt werden: Ruthenium(II)-(tris-4,7-diphenyl-1,10-phenanthrolin)/HPTS Ruthenium(II)-(tris-4,7-diphenyl-1,10-phenanthrolin)/Fluorescein Ruthenium(II)-(tris-4,7-diphenyl-1,10-phenanthrolin)/Rhodamin B Ruthenium(II)-(tris-4,7-diphenyl-1,10-phenanthrolin)/Rhodamin B-octadecylester Ruthenium(II)-(tris-4,7-diphenyl-1,10-phenanthrolin)/Hexadecyl-Acridinorange Europium(III)-tris-theonyl-trifluormethylacetonat/Hydroxymethylcoumarin Platin(II)-tetraphenylporphyrin/Rhodamin B-octadecylester Platin(II)-tetraphenylporphyrin/Rhodamin B Platin(II)-tetraphenylporphyrin/Naphtofluorescein Platin(II)-tetraphenylporphyrin/Sulforhodamin 101 Platin(II)-octaethylporphyrin/Eosin Platin(II)-octaethylporphyrin/Thionin Platin(II)-octaethylketoporphyrin/Nilblau Cr(III)-YAG/Nilblau und Cr(III)-YAG/Naphtofluorescein. Aminocoumarin/Aminofluorescein Aminocoumarin/Rhodamin 6G Aminocoumarin/Tetramethylrhodamin Aminocoumarin/Acridinorange Aminofluorescein/Rhodamin 6G Aminocumarin/Nilblau Aminofluorescein/Tetramethylrhodamin Aminofluorescein/Ethidiumbromid und DAPI/Rhodamin.

Es können aber auch Kombinationen mit 3 Frabstoffen zur Kodierung der Partikel zum Einsatz kommen wie z.B.:
Aminocumarin, Rhodamin, Nilblau oder
Aminocumarin, Fluorescein, Rhodamin. Weitere Kombinationen sind möglich.

Als Oberflächenfluoreszenz kann entweder die Fluoreszenz der äußersten Schicht der Mikropartikel bezeichnet werden oder, wenn die Akzeptormoleküle auf der Partikeloberfläche selbst fluoreszenzmarkiert sind, die Fluoreszenz dieser Akzeptormoleküle.

Als Ligandenfluoreszenz wird die Fluoreszenz bezeichnet, die entweder die von mit einem entsprechenden Farbstoff markierten Analyten stammt oder, wenn ein entsprechend markierter konkurrierender Ligand eingesetzt wird, die Fluoreszenz dieses markierten Liganden oder wenn ein fluoreszierendes Detektionsmolekül an den an die Akzeptormoleküle gebunden Liganden bindet.

In einer bevorzugten Variante werden für die Partikelkodierung ein oder mehrere Farbstoffe der Klassen Cumarine, Rhodamine oder Nilblauderivate in die Partikel einpolymerisiert und ein Fluoresceinderivat als Oberflächenfluoreszenz bzw. für die Markierung von Liganden.

Wird mit Cumarin, Fluorescein und Rhodamin kodiert, können bevorzugt Cy5 und Fluoreszenzfarbstoffe höherer Emmissionswellenlänge für die Oberflächenfluoreszenz bzw. für die Ligandenfluoreszenz genutzt werden. Es ist aber auch möglich, die Oberflächenfluoreszenz bzw. Ligandenfluoreszenz über ein FRET-Paar zu realisierten wie z.B. Fluorescein und Rhodamin. Dann stehen für die Kodierung Cumarin- und Cyanfarbstoffe etc. zur Verfügung. Die Kodierungsmöglichkeiten erhöhen sich dadurch enorm, dass ein und derselbe Farbstoff in mehreren Schichten der Mikropartikel in unterschiedlichen Konzentrationen eingesetzt werden kann. Dadurch resultiert für jede Schicht und zwischen verschiedenen Schichten eine Vielzahl von Ratio-Bildungsmöglichkeiten, die für die Dekodierung der Partikelpopulationen genutzt werden können.

Eine weitere Möglichkeit der Strukturbildung innerhalb de Mikropartikel besteht in der schichtweisen Einpolymerisation von weiteren fluoreszierenden oder fluoreszenzkodierten Mikropartikeln. Dadurch können in den Mikropartikeln gezielt Muster erzeugt werden wie z.B. grob oder feingranuliert oder die Zahl der Granula pro Schicht, die für die Kodierung zahlreicherer Populationen von Mikropartikeln genutzt werden können.

Gemäß der Erfindung werden bevorzugt Mikropartikel aus Melamin, Silika, Polysulfon und Polyether, Polymethacrylat, ganz bevorzugt aus Polymethacrylat eingesetzt. Sie haben i.d.R. einen Durchmesser von 0,5 - 60 µm. Die bevorzugte Partikelgröße beträgt 5-15 µm. Die Form der Partikel kann dabei sphärisch, elliptisch, zylindrisch, unregelmäßig oder kuboid sein.

Die vorzugsweise verwendeten Mikropartikel zeichnen sich dadurch aus, dass sie bevorzugt aus 2 Schichten und einem Kern bestehen, die sich fluoreszenzoptisch unterscheiden und wobei wenigstens die äußere Schicht temperaturbeständig ist und z.B. aus Melamin besteht. Die mittlere und innere Schicht bestehen bevorzugt ebenfalls aus temperaturbeständigem Polymer, sofern die eingesetzten Tests bei höheren Temperaturen ablaufen. Die Partikel können in einer oder mehreren Schichten Magnetpartikel enthalten, mit deren Hilfe eine sichere Immobilisierung der Partikel während der Messung gewährleistet ist.

Die verschiedenen Schichten können einen oder mehrere Fluoreszenzfarbstoffe enthalten, die für die Kodierung genutzt werden ebenso wie Fluoreszenzfarbstoffe, die direkt oder vermittelt über Biomoleküle an die Partikeloberfläche gekoppelt wurden. Fluoreszenzfarbstoffe, die über Biomoleküle an die Oberfläche gebunden wurden können somit die Funktion Kodierung als auch die Funktion der Indikation der Bindungs- oder Reaktionskinetik haben.

Alternativ zu fluoreszenzkodierten Partikeln können auch Zellen in Form von Suspensionen, Monolayer oder Gewebeschnitten eingesetzt werden, wobei unterschiedliche Zellpopulationen über ihre Feinstruktur oder gegebenenfalls über ihre Fluoreszenzkodierung voneinander differenziert werden können.

Die Polymere an der Partikeloberfläche können in unmodifizierter Form oder in modifizierter Form vorliegen. Mögliche Funktionalisierungen sind Carboxy-, Sulfohydryl-, Epoxy-, Amino-, Hydroxy-, Sulfonsäure-, Pegylyl-, Acryl-, Phosphat-Gruppen sein.

### Mikropartikelimmobilisierung

Bevorzugt wird eine Vorrichtung verwendet, die als Messpunkte fluoreszenzkodierte Mikropartikel beinhaltet, die die Akzeptormoleküle tragen und durch Schwerkraft, magnetische oder mechanische Kräfte, durch chemische oder physikalische Bindung am Gefäßboden permanent oder während der Dauer des Messvorganges immobilisiert sind. Dabei können die Mikropartikel direkt an den Träger oder vermittelt über Linkermoleküle gekoppelt werden. Als Linkermoleküle fungieren auch die immobilisierten Akzeptormoleküle die bevorzugt Biomoleküle darstellen. Die immobilisierten Partikel der verschiedenen Partikelpopulationen verteilen sich zufällig über das vorgesehene Areal des Trägers und können als Singletten, Dubletten, Tripletten, Multipletten, als komplexe Arrangements und sogar als Monolayer vorliegen. Die wichtigste Funktion der Partikelimmobilisierung ist der Vorteil, dass die Dekodierung der Partikelfluoreszenzen nur einmal zu erfolgen hat und danach nur noch die Oberflächen bzw. Ligandenfluoreszenz für die Aufzeichnung weiterer Messpunkte erfasst werden muss. Werden die Partikel nicht permanent immobilisiert, hat die Aufnahme der Partikelfluoreszenzen auch während der Aufzeichnung der weiteren Messpunkte zu erfolgen.

Die Partikel müssen nicht fest immobilisiert an einer festen Oberfläche sondern stabil in einer Fokusebene gehalten werden, was auch durch einen Dichtegradienten gewährleistet werden kann. Bei Verwendung von Partikeln unterschiedlicher Dichten, kann somit eine zusätzliche Kodierung von Partikelpopulationen erzielt werden. Unterschiedliche Dichten können z.B. durch Verwendung unterschiedlicher Polymere bei der Herstellung von Partikeln und Partikelschichten eingesetzt werden. Der Aufbau des Gradienten wird z.B. durch simultanes Pipettieren von Probe und Dichtegradienten gewährleistet, wobei die Verdünnung der Probe gleich sein oder über den Gradienten variieren kann. Bei Variation der Probenverdünnung ist es möglich, in einem Reaktionsansatz z.B. eine Titrierung des Analyten vorzunehmen. Der Dichtegradient kann als homogener Gradient oder als Stufengradient vorliegen. Die Partikel können durch Schwerkraft an die gewünschte Phasengrenze sedimentieren. Handelt es sich um einen Ladungsgradienten der bevorzugt durhc Ampholyte eingestellt wird, können die Partikel auch entsprechend ihrer Ladung durch ein angelegtes Feld zu ihrem isoelektrischen Punkt migrieren. Durch Kombination homogener Ladungs- und Dichtegradienten können Bead-Multilayern im Reaktionsgefäß erstellt werden.

Von Vorteil ist, daß die Phasengrenzen zwischen unterschiedlichen Dichten auch bei nichtplanaren Böden der Reaktionsumgebung, Böden von Mikrotestplatten sind z.B. nie ideal planar, trotzdem eine planare Fokusebene erreicht wird. Dadurch wird Fokussieraufwand und somit Zeit gespart. Es ist ebenso möglich, die Partikel mit der Reaktionslösung zwischen einer weniger dichten apolaren und einer dichteren apolaren Flüssigkeit einzuschließen, um auf diese Weise den Reaktionsraum zu begrenzen. Dadurch ist es auch möglich, die Hintergrundfluoreszenz zu minimieren. Hintergrundfluoreszenzen kann auch dadurch minimiert werden, dass der Reaktionslösung z.B. ein Quencherfarbstoff oder lichtabsorbierende Nanopartikel zugesetzt werden, die die Reaktion nicht beeinträchtigen aber Kontrast und somit Sensitivität erhöhen. Bevorzugt werden solche Stoffe zur Kontrasterhöhung zugesetzt, die Licht der Anregungswellenlänge und / oder der Emmissionswellenlänge des oder der Ligandenfluoreszenz entsprechen.

### Akzeptormoleküle

Als Akzeptormoleküle, die bevorzugt Biomoleküle sind, werden solche Stoffe eingesetzt, die spezifisch den Analyten aus der Probe oder der Referenzprobe mit hoher Sensitivität und Spezifität binden oder die mit dem Analyten um die Bindung eines Analogons konkurrieren. Akzeptormolekül und Analogon bzw. Ligand können eine Fluoreszenzmarkierung und / oder einen Löscher (Quencher) tragen. Die immobilisierten Biomoleküle können Peptide oder Proteine und hier bevorzugt Antikörper, Antigene, Enzyme, Lektine, Aptamere sein. Sie können aber auch Polysaccharide, Lipide oder Nukleinsäuren sein. Im Falle von Nukleinsäuren werden meist synthetische DNA- oder RNA-Oligonukleotide eingesetzt. Es können aber auch nicht natürliche Nukleinsäureanaloga zur Anwendung kommen.

Die Kopplung der Akzeptormoleküle an die Mikropartikel erfolgt kovalent und oder nichtkovalent.

### Fluoreszenzänderung

Die für die Detektion notwendigen Reaktionen sind intermolekulare Wechselwirkungen, die an der Grenzfläche zwischen der festen Phase der Partikeloberfläche und der flüssigen Phase der Reaktionslösung ablaufen. Dabei wird in Abhängigkeit des Testprinzips eine Fluoreszenzänderung der Oberflächen- bzw. Ligandenfluoreszenz verursacht die sich durch eine Zunahme oder Abnahme der Oberflächen- oder Ligandenfluoreszenzintensität, -polarisation, -lebensdauer an der Partikeloberfläche bemerkbar macht und im Zeitverlauf mit mindestens zwei Messpunkten aufgezeichnet wird. Eine Verringerung der Fluoreszenzintensität kann eintreten, indem, wie bereits oben geschildert, auf der Partikeloberfläche immobilisierte fluoreszenzmarkierte Biomoleküle in der Reaktion degradiert werden.

Alternativ kann es zu einer Fluoreszenzsignalerhöhung kommen, wenn ein Quencher durch die Reaktion aus der räumlichen Nähe zum gequenchten Fluorophor gedrängt wird, wobei das Fluorophor wie z.B. im Falle von Molecular Beacons, Scorpions, Amplifluor-Primern zu fluoreszieren beginnt. Das Quenching des Fluorophors kann auch durch Degradation einer immobilisierten TaqMan-Sonde während der Reaktion aufgehoben werden, wobei der Fluorophor nach Degradation der Sonde an der Partikeloberfläche verbleibt und fluoresziert. Ebenfalls ist es möglich, das FRET-Paare durch die räumliche benachbarte Bindung von mit Fluoreszenzfarbstoffmolekülen markierten Sonden an die Analytmoleküle entstehen, die ihrerseits während der Reaktion auf der Oberfläche der Partikel z.B. durch PCR immobilisiert wurden. Es ist auch vorgesehen, das eine Erhöhung der Fluoreszenzsignale der Ligandenfluoreszenz auf der Partikeloberfläche dadurch entstehen kann, dass fluoreszenzmarkierte Detektionsmoleküle wie z.B. Anti-Phosphatgruppen-spezifische Antikörper binden. Durch diese Bindung an während der Reaktion phosphorylierten und auf der Partikeloberfläche immobilisierten Peptiden werden fluoreszierende Gruppen in ihrer Konzentration messbar erhöht, ohne das Quenching und FRET-Systeme benötigt werden.

Die zu messende Fluoreszenzänderung kann bevorzugt:
- in einer Abnahme der Oberflächenfluoreszenz bestehen, z.B. durch Degradation.
- in einer Zunahme der Oberflächenfluoreszenz bestehen, z.B. durch Verdrängung eines mit einem Quencher markierten konkurrierenden Liganden vom mit einem entsprechenden Farbstoff markierten Akzeptormolekül durch einen oder mehrere Analyte.
- in einer Abnahme der Ligandenfluoreszenz bestehen, z.B. durch spezifische Bindung eines mit einem entsprechenden Farbstoff markierten Analyten an einen mit einem Quencher markierten Akzeptor auf der Partikeloberfläche.
- in einer Zunahme der Ligandenfluoreszenz bestehen, z.B. durch spezifische Bindung eines mit einem entsprechenden Farbstoff markierten Analyten an ein entsprechendes Akzeptormolekül auf der Mikropartikeloberfläche.
- in einer Veränderung der Gesamtfluoreszenz bestehen, z.B. durch Zusammenspiel eines FRET-Paares zwischen Akzeptormolekül und Analyt.

Beispiele für Reaktion sind PCR, Festphasen-PCR, isothermische Nukleinsäureamplifikationen wie "Strand Displacement Amplification" oder "Ligase Chain Reaction" oder andere Enzymreaktionen, Bindungen von Antikörpern an Antigene.

### Messsystem

Geräte zur erfindungsgemäßen Durchführung und Messung von Fluoreszenzänderungen am Beadarray verschiedenster Testformate, die vollautomatisch mittels Computer steuerbar sind, umfassen:
- eine vollautomatische Steuerung mittels Computer,
- einen Thermocycler für die schnelle Temperierung der Proben, mit einer Heiz-/Kühlrate von 3-20 °C pro Sekunde, welcher einen Reaktionsbereich mit mehreren temperierbaren Aufnahmen für Träger gemäß Anspruch 1, Schritt a) aufweist,
- eine Positioniereinrichtung für den Thermocycler und die Reaktionsumgebung die thermozyklisch steuerbar ist,
- eine dem Reaktionsbereich zugeordnete Beleuchtungseinrichtung mit denen Anregungslicht unterschiedlicher, definierten Wellenlängen strahlbar ist,
- eine Optikeinrichtunge die vorzugsweise für die everse und inverse Fluoreszenzdetektion mit entsprechenden optischen Filtern geeignet ist,
- eine oder mehrere Detektoreinrichtung(en) (z.B. CCD, CMOS), die in Abhängigkeit von einer gemessenen Fluoreszenzintensität Bilder erzeugt, und
- eine Auswerteeinheit, die aus den Bildern Messwerte erzeugt.

Für den Einsatz von Mikropartikeln können die Geräte zusätzlich Immobilisierungseinrichtungen für z.B. magnetische oder paramagnetische Partikel umfassen.

Durch die Verwendung optisch transparenter Gefäßboden und/oder Gefäßdeckel mit geringer Eigenfluoreszenz, welche die fluoreszenzoptische Auswertung während der thermozyklischen oder isothermischen Durchführung der Nachweisreaktion ermöglichen, sind Messungen mit Auflicht und Durchlichtfluoreszenz möglich. Bei Auflichtfluoreszenz können handelsübliche Mikrotiterplatten eingesetzt werden wie z.B. NucleoLink oder NucleoSorb. Die verwendeten handelsüblichen Reaktionsgefäße können zur Verringerung der Eigenfluoreszenz beschichtet werden.

Da bei Einsatz nicht permanent immobilisierter Mikropartikeln alle Partikelfluoreszenzen während der Aufnahme weiterer Messpunkte gemessen werden müssen, sind besonders hohe Anforderungen an die Schnelligkeit der Hardware gestellt wie z.B. die Verwendung möglichst geringer Auflösung der Objektive bei hoher Lichtstärke, die Verwendung von Multibandfiltern zur Vermeidung von Filterwechseln in Kombination mit einer Mehrfarben-LED-Beleuchtung, Verwendung einer elektronenverstärkenden Kamera, Parallelisierung der Detektoreinheiten. Detektoreinheiten können im Sinne einer Parallelisierung bevorzugt in 8er-Zeilen angeordnet sein oder durch Glasfaser- oder Flüssigleitern auf eine Kamera gebündelt werden. Ebenfalls ist es möglich durch Weitwinkelobjektive mehrere Wells gleichzeitig zu messen. Durch Pixelbinning läßt sich die Messzeit ebenfalls reduzieren, da die Sensitivität steigt, was die Belichtung verkürzt, und die Datenmenge reduziert wird.

### Aufnahme von Messpunkten

Die Erfindung sieht vor, dass vor oder zu Beginn der Reaktion ein erster Messpunkt und im Verlauf oder wenigstens nach Abschluss der Reaktion ein weiterer Messpunkt aufgezeichnet werden, um berechnen zu können wie stark die Reaktion für den einzelnen Analyten verlief oder in welcher Konzentration der Analyt in der Probe vorliegt.

Die Aufnahme des ersten Messpunktes ist erforderlich, um alle für die Auswertung in Betracht kommenden Mikropartikel zu identifizieren indem die Kodierungsfluoreszenzen erfasst werden, um alle Partikel einer Partikelpopulation zuordnen zu können. Außerdem wird die für die Auswertung erforderliche x/y-Position einer notwendigen Anzahl identifizierter Partikel pro Population abgespeichert, um die Messorte für die Aufzeichnung der Oberflächen bzw. Ligandenfluoreszenz bei der Erfassung weiterer Messpunkte wiederzufinden. Des Weiteren wird die Oberflächen- bzw. Ligandenfluoreszenz als Referenz für das in den folgenden Messpunkten aufgezeichnete Oberflächen- bzw. Ligandensignal erfasst und gegebenenfalls auch zur Dekodierung der Partikelpopulation eingesetzt. Die Aufnahmen der Fluoreszenzen erfolgen mit der optischen Auflösung 1, die in der Regel höher als Auflösung 2 ist, um sicher die auswertbaren Einheiten, bevorzugt einzeln immobilisierte Partikel aber gegebenenfalls auch Dubletten, Tripletten oder Monolayern von Partikeln zu erfassen. Dies kann bevorzugt dadurch erreicht werden, dass durch Aufnahme von Bildern in mehreren Z-Ebenen und der anschließenden Berechnung eines Gesamtschärfebildes ("extended-depth-of-field"-Algorithmen) kann aus Doubletten und höheren Agglomeraten die Einzelbeadgrenze sicher bestimmt und damit eine Vereinzelung erreicht werden.

Alternativ werden erkannte Agglomerate anhand ihrer Fläche und Morphologischen Eigenschaften in einzelne Gruppen unterteilt. Für jede Gruppe wird eine spezielle Algorithmusvariante zur Berechnung der Fluoreszenz verwendet. Über die Ermittlung der Bead-Berührungspunkte können die Beads digital vereinzelt werden.

Alternativ können über Beadextrapolation durch Verwendung von a-priori Wissen (Bead ist z.B. eine Kugel) die Ringstrukturen im Bild ergänzt werden, die von anderen Beads überlagert sind. Dadurch können Mehrschichtanordnungen von Beads ausgewertet werden. Ein Algorithmus aus der Literatur ist die Hough-Circle-Transformation) Ausgewertet wird immer die von einem Bead sichtbare Fläche.

Alternativ werden durch digitale Löschung einzelner Beadpopulationen aus den Bilddaten aus Beadagglomerate Einzelbeads extrahiert. Diese Alternativen können einzeln oder in Kombination angewendet werden, wodurch mehr auswertbare Partikel pro Bildaufnahme zur Verfügung stehen, was wiederum Zeit bei der Analyse spart.

Die Erfassung aller weiteren Messpunkte erfolgt mit Auflösung 2 und beschränkt sich bevorzugt nur auf die Erfassung der Oberflächen- bzw. Ligandenfluoreszenz. In einer weiteren Ausführungsvariante ist es möglich, auch Partikelfluoreszenzen zu erfassen, um wenigsten für einige Messpunkte die genaue Wiederfindung der Partikel zu dokumentieren. Strahlt eine Fluoreszenz der Partikelfluoreszenz geringfügig aber sicher detektierbar in den Emissionskanal der Oberflächenfluoreszenz, reduziert dies zwar die Empfindlichkeit aber der Partikel kann wiedergefunden werden. Es ist ebenfalls möglich, zwei Oberflächenfluoreszenzen einzusetzen, wobei eine Fluoreszenz keiner Fluoreszenzänderung während der Reaktion unterliegt, so dass eine Partikelverschiebung auf Grund z.B. thermischer Effekte ausgeschlossen werden kann. Die verwendeten Auflösungen sind optimal auf die Partikeldichte und die Zahl der Pixel pro Partikel (1-1000 Pixel) anzupassen, um die für die jeweilige Anwendung benötigte Sicherheit bei der Identifizierung und Wiederfindung der Partikel zu gewährleisten.

Die Aufzeichnung der weiteren Messpunkte kann in regelmäßigen Zeitabständen vor, während und nach der Reaktion erfolgen oder aber nachdem durch die Temperierung eine bestimmte Temperatur oder ein bestimmter Abschnitt eines Thermozyklus erreicht wurde. Bevorzugt erfolgen die Messungen während eines isothermischen Abschnittes der Reaktion, um Einflüsse die durch Temperaturschwankungen wie Materialdehnung und verstärkte Konvektion im Reaktionsgemisch zu minimieren.

Es kann von Vorteil sein, von Zeit zu Zeit neben der Aufnahme der Oberflächen bzw. Ligandenfluoreszenz auch eine Partikelfluoreszenz aufzunehmen, da bei abnehmender Oberflächenfluoreszenz die Gefahr besteht, dass ein Partikel nicht korrekt wiedergefunden wird. Um Bleaching zu minimieren, kann bevorzugt bei Verwednung von LED insbesondere bei der Aufzeichnung weiterer Meßpunkte eine Anpassung der Beleuchtungsstärke auf die Oberflächenfluoreszenz vorgenommen werden. Die variierte Beleuchtungsstärke ist gegebenenfalls zu referenzieren. Reduktion des Bleachings durch Begrenzung der Beleuchtung auf die Zeit der Bildaufnahme. Außerdem kann durch die Verringerung der Objektivvergrößerung eine größere Messfläche abgebildet werden, wodurch weniger Bilder pro Well benötigt werden.

Die Vermeidung von Nachfokussierung und Nachbelichtung durch Algorithmen wie "extended-depth-of-field" (Erhöhung der Tiefenschärfe durch Zusammenrechnen mehrerer Bilder in Z) und "high-dynamic-range" (Erhöhung der Belichtungsdynamik durch Zusammenrechnen eines sehr hellen und eines sehr dunklen Bildes) verkürzen ebenfalls die Messzeit.

Pro Messpunkt werden 1-1000 Partikel einer Population erfaßt, wobei bevorzugt 1-100 Partikel und in der besonders bevorzugten Ausführungsvariante 1-10 Partikel erfaßt werden.

### Analyse

### Zuordnung der Messpunkte zu Partikel und Partikelpopulationen

Bevorzugt werden zur Dekodierung der Partikelpopulationen die Fluoreszenzen der in die Partikel insgesamt oder in die verschiedenen Schichten einpolymerisierten Farbstoffe eingesetzt. Aus den verschiedenen z.B. Fluoreszenzintensitäten werden Verhältnisse (Ratios) berechnet, die für jede Partikelpopulation eine eindeutige Kennzeichnung erlauben.

So kann die Fluoreszenz in Form der Bestimmung der Fluoreszenzintensität einer bestimmten Emissionswellenlänge, in der Partikelperipherie als Ringfluoreszenz erkennbar, vor dem Start einer Nukleasereaktion zur Bestimmung der Partikelpopulation aber auch zur Bestimmung des Maximalsignals der Oberflächenfluoreszenz des betreffenden Partikels vor der Reaktion bestimmt werden. Im Verlauf der Reaktion nimmt dieses Maximalsignal ab, da die Biomoleküle enzymatisch degradiert werden und somit der Farbstoff von der Partikeloberfläche freigesetzt wird. Für die Dekodierung der Partikelpopulationen steht der Farbstoff dann zwar nicht mehr zur Verfügung. Da der Partikel immobilisiert wurde, braucht die Dekodierung aber auch nicht mehr erfolgen. Zur Kontrolle der korrekten Partikelwiederfindung ohne Dekodierung sind in diesem Falle die Fluoreszenzen der in die Partikel einpolymerisierten Farbstoffe selbst ausreichend. Kodierungs-, Oberflächen- und/oder Ligandenfluoreszenz können ebenfalls dazu herangezogen werden, um Partikel unterschiedlicher Größen in Bezug auf detektierte Fluoreszenzintensitäten zu referenzieren.

### Errechnen von Kinetiken

Durch die Analyse der Fluoreszenzänderung als Folge der Reaktion von Messpunkt zu Messpunkt ergeben sich Reaktionskinetiken aus denen sich Konzentrationen der Analyten oder auch Bindungskonstanten errechnen lassen. Erfolgt die Auswertung bezogen auf einen Reaktionszyklus wie bei der Polymerase-Kettenreaktion ist die Angabe des Durchbruchszyklus, bei dem das Signal oder die Signaländerung den Hintergrund durchbricht als Maß für die gebildete Menge an Reaktionsprodukt entscheidend. Auf diese Weise ist eine viel genauere Quantifizierung der PCR-Produkte in einer Probe möglich als durch die Bestimmung der Fluoreszenzintensität.

Durch die Referenzierung aller weiteren Messpunkte auf Messpunkt 1 oder einen der darauffolgenden Messpunkte vor oder zu Beginn der Reaktion werden Messfehler deutlich reduziert, da die mögliche Referenzierung auf eine Partikelpopulation als Positivkontrolle oder auf eine Referenzprobe Einflüsse wie unterschiedliche Ausleuchtung oder Partikelgröße minimiert werden können.

Das Verfahren sieht ebenfalls vor, das der Einfluss von Störgrößen systematisch erfasst werden muss, um diese bei der Berechnung der Messwerte berücksichtigen zu können. Solche Störgrößen können das Ausbleichen (Bleaching/Fading) einiger Fluoreszenzfarbstoffe sein ebenso wie Einflüsse von Temperaturschwankungen auf die Messung oder die unspezifische Hintergrundfluoreszenz.

### Kit

Es wird auch ein Kit zur Verwendung in einem erfindungsgemäßen Verfahren offenbart, wobei der Kit vorformulierte Reagenzien enthält, die mindestens eine mit Akzeptormolekülen beschichtete Mikropartikelpopulation umfassen.

Mit dem erfindungsgemäßen System kann ein vorgefertigter Realtime-PCR-Kit bereitgestellt werden, der alle nötigen Ingredienzien enthält. Damit ist das einzelne Pipettieren von Puffer, Nukleotidmix, Reportermoleküle, Polymerasen, Reverse Transkriptasen und Primer sowie gegebenenfalls von Anti-Bleaching-Mitteln nicht notwendig. Für die Quantifizierung wird bei diesem (klassischen) Verfahren der Realtime-PCR die Schmelzpunktbestimmung der amplifizierten DNA und die Analyse des Schwellenwertes der Fluoreszenzintensität (CT) genutzt.

Das erfindungsgemäße Verfahren kann verwendet werden für:
- eine qualitative und quantitative Nukleinsäure-Multiplexamplifikation,
- ein Nukleinsäure-Sequenzscreening,
- eine Immundetektion zur Bestimmung der Konzentration von Antigenen und Antikörpern bzw. entsprechender Bindungskonstanten und der Bestimmung von Enzymaktivitäten sowie von intermolekularen Wechselwirkungen zwischen Proteinen, Proteinen und niedermolekularen Substanzen, Proteinen und Nukleinsäuren, Kohlenhydraten, Lipiden.

Die vorliegende Erfindung bezieht sich auch auf:
- ein erfindungsgemäßes Verfahren, dadurch gekennzeichnet, dass der Träger thermostabil ist.
- ein erfindungsgemäßes Verfahren, dadurch gekennzeichnet, dass der Träger transparent ist.
- ein erfindungsgemäßes Verfahren, dadurch gekennzeichnet, dass die planaren Bereiche des Trägers beschichtet sind, um die Immobilisierung der Mikropartikel zu verbessern oder unspezifische Hintergrundreaktionen zu verringern.
- ein erfindungsgemäßes Verfahren, dadurch gekennzeichnet, dass die Schichten der Mikropartikel weitere fluoreszierende, fluoreszenzkodierte oder magnetische Mikro- oder Nanopatikel enthalten.
- ein erfindungsgemäßes Verfahren, dadurch gekennzeichnet, dass als Fluoreszenzfarbstoffe bevorzugt Cumarine, Fluoresceine, Rhodamine, Cyananine angewendet werden.
- ein erfindungsgemäßes Verfahren, dadurch gekennzeichnet, dass die Mikropartikel bis mindestens 95° C thermostabil sind.
- ein erfindungsgemäßes Verfahren, dadurch gekennzeichnet, dass die Mikropartikel Oberflächenfunktionalisierungen aufweisen, die für die kovalente oder nicht kovalente Kopplung von Akzeptormolekülen und die Immobilisierung der Partikel am Träger genutzt werden können.
- ein erfindungsgemäßes Verfahren, dadurch gekennzeichnet, dass die Mikropartikel bevorzugt aus Polystyrol, Polymethacrylat, Polysulfon, Polyether, Silika oder Melaminharz bestehen.
- ein erfindungsgemäßes Verfahren, dadurch gekennzeichnet, dass die fluoreszenzkodierten Mikropartikel durch Schwerkraft, magnetische, elektrische Kräfte oder durch chemische oder physikalische Bindung, bzw. teilweise Einbettung in einem Polymer am Gefäßboden oder in einem Dichte- und/oder Ladungsgradienten permanent oder während der Dauer des Messvorganges immobilisiert sind.
- ein erfindungsgemäßes Verfahren, dadurch gekennzeichnet, dass die fluoreszenzkodierten Mikropartikel vermittelt über Linker- oder Akzeptormoleküle am Träger immobilisiert sind.
- ein erfindungsgemäßes Verfahren, dadurch gekennzeichnet, dass die Akzeptormoleküle die oder den spezifisch Analyten aus der Probe oder der Referenzprobe mit hoher Sensitivität und Spezifität binden oder daß die Akzepormoleküle mit dem Analyten um die Bindung eines Analogons konkurrieren.
- ein erfindungsgemäßes Verfahren, dadurch gekennzeichnet, dass als Akzeptormoleküle Biomoleküle und bevorzugt Peptide, Proteine, Antikörper, Antigene, Enzyme, Lektine, Aptamere, Polysaccharide, Lipide, Glycolipide, Hormone, Nukleinsäuren oder Nukleinsäureanaloga sein können.
- ein erfindungsgemäßes Verfahren, dadurch gekennzeichnet, dass die Interaktion zwischen Akzeptormolekülen und Analyten bzw. Analogon zu einer Fluoreszenzänderung im Sinner einer Verringerung oder Zunahme einer Fluoreszenz führt.
- ein erfindungsgemäßes Verfahren, dadurch gekennzeichnet, dass ein Messgerät verwendet wird, bei welchem der Termocycler mit einem Elektromagneten oder permanenten Magneten zur Immobilisierung der Mikropartikel kombiniert ist.
- ein erfindungsgemäßes Verfahren, dadurch gekennzeichnet, daß besonders bevorzugt 1-10 Pixel pro vorselektierten Partikel aufgezeichnet werden.
- ein erfindungsgemäßes Verfahren, dadurch gekennzeichnet, daß wenigstens ein Pixel den vorselektierten Partikel erfaßt.
- ein erfindungsgemäßes Verfahren, dadurch gekennzeichnet, dass ausschließlich als Singletten, Dubletten, Tripletten und / oder als Verteilungen höherer Ordnung einschließlich als Monolayern verteilte Partikel berücksichtigt werden.
- ein erfindungsgemäßes Verfahren, dadurch gekennzeichnet, dass die weiteren Messpunkte nach Inkontaktbringen gemäß Schritt c) entsprechend vordefinierter Zeitabstände oder nach dem Erreichen definierter Reaktionstemperaturen erfolgt.
- ein erfindungsgemäßes Verfahren, dadurch gekennzeichnet, dass die im Zeitverlauf oder in unterschiedlichen Reaktionszyklen aufgezeichneten Messwerte der weiteren Messpunkte einer Messreihe ein und des selben Messortes auf einen oder mehrere Messwerte derselben Messreihe, desselben Messortes oder auch anderer Messorte zu referenzieren.
- ein erfindungsgemäßes Verfahren, dadurch gekennzeichnet, dass die Meßwerte unter Einbeziehung vorab bestimmter Bleaching-Zeitverläufe korrigiert werden.
- die Verwendung eines erfindungsgemäßen Verfahrens zur Bestimmung der Reaktionskinetik für mindestens einen Analyten in der zu analysierenden Probe.

### Vorteile:

- Referenzierung nur auf Messpunkt 1 und ggf. auf eine Vergleichsprobe. Dadurch wird die Messung genauer und es können weniger Partikel pro Population eingesetzt oder vermessen werden.
- nur einmalige Ortsbestimmung und Dekodierung notwendig. Dadurch kann Zeit eingespart werden und eine Datenreduktion erreicht werden.
- geringere Auflösung 2 trägt dazu bei, dass ein großes Areal aufgezeichnet werden kann und deshalb mehr Partikel pro Aufzeichnungsvorgang erfasst werden, was Zeit spart.
- Ein Mix & Measure-Assay ist einfach in der Handhabung, so dass wenige Fehlermöglichkeiten resultieren. Außerdem ist es möglich Reaktionskinetiken zu erfassen, wodurch die Qualität der Analyse erweitert wird.
- Die Reduzierung der für die Auswertung benötigten Anzahl von Partikeln pro Population ermöglicht nicht nur eine Material- und Zeitersparnis, sondern auch die Messung von mehr Populationen pro Flächeneinheit wodurch der mögliche Multiplexgrad steigt.
- Durch Verwendung von 2 unterschiedlichen Auflösungen ist es möglich vor dem Start der Reaktion durch eine höhere Auflösung alle auswertbaren Partikel zu identifizieren und zu lokalisieren, was zeitaufwändig ist. Für die Erfassung der Oberflächenfluoreszenz bzw. der Ligandenfluoreszenz anderer Messpunkte kann mit Auflösung 2 gearbeitet werden, da bereits nur die Messpunkte mit Auflösung 1 identifiziert wurden, die sicher mit Auflösung 2 bei der Erfassung späterer Messpunkte wiedergefunden werden. Da mit der geringeren Auflösung 2 mehr Partikel in einem größeren Messfeld auf dem Träger erfasst werden können, wird der Messprozess erheblich beschleunigt.
- Da bei der Aufzeichnung weiterer Meßpunkte für nur eine Fluoreszenzemission der Oberflächen bzw. Ligandenfluoreszenz keine Filterwechsel nötig sind, wird Zeit eingespart.
- Im Vergleich zu konventionellen Testsystemen bewirken die zeitliche und räumliche Dimensionierung der Detektion der verschiedenen Parameter zu einer großen Robustheit des Tests.

Der Kern enthält z.B. 100 Teile Aminocumarin und z.B. 100 Teile Nilblau (100/100). Die mittlere Schicht enthält z.B. 100 Teile Fluorescein und die äußere Schicht enthält z.B. 50 Teile Aminocumarin und z.B, 100 Teile Nilblau.

Partikel aus Partikelpopulation 2 (rechte Abbildungshälfte):

Der Kern enthält z.B. 100 Teile Aminocumarin und z.B. 100 Teile Nilblau (100/100). Die mittlere Schicht enthält z.B. 100 Teile Fluorescein und die äußere Schicht enthält z.B. 100 Teile Aminocumarin und z.B. 100 Teile Nilblau.

### Farbstoff 1 am Anti-Phosphogruppen-Antikörper ist z.B. Fluorescein oder Rhodamin.

Die Partikel von mit Peptiden beschichteten Mikropartikelpopulationen und das Reaktionsgemisch bestehend aus Pufferlösungen und der Anti-Phosphogruppen-Antikörper werden in die Kavität einer Mikrotestplatte gegeben. Alle Mikropartikel im Ansatz werden sedimentiert oder durch einen permanenten oder elektrischen Magneten am Gefäßboden immobilisiert, sofern sie nicht bereits vorher fest am Boden der Mikrotestplatte immobilisiert wurden.

Durch Fluoreszenzspektroskopie werden alle Fluoreszenzen der Mikropartikelpopulationen im Rahmen von Messpunkt 1 aufgezeichnet und die Mikropopulationen identifiziert sowie die Lage der Partikel im Reaktionsgefäß bestimmt. Dazu erfolgt die Anregung des beispielhaft genannten Aminocumarins bei einer Wellenlänge von 350 nm, um die erste Kodierungsfluoreszenz der Partikel in der äußeren und in der inneren Schicht zu erfassen. Anschließend wird z.B. Fluorescein bei einer Wellenlänge von 480 nm angeregt und bei 520 nm gemessen, um die zweite Kodierungsfluoreszenz der mittleren Schicht der Partikel und die Ligandenfluoreszenz zu bestimmen. Durch nachfolgende Anregung bei 680 nm und Detektion bei 720 nm wird das beispielhaft genannte Nilblau angeregt und detektiert, um die zweite Kodierungsfluoreszenz des Kerns bzw. der äußeren Schicht zu bestimmen.

Die Verrechnung der ortsaufgelöst detektierten Fluoreszenzsiganle erfolgt nach folgenden Regeln:

### Kern:

- Ermittlung des Quotienten 1 aus Aminocumarin- und Nilblau-Fluoreszenz
- Ermittlung des Kerndurchmessers

### Mittlere Schicht:

- Ermittlung des Quotienten 2 aus Fluoresceinfluoreszenz und Aminocumarin- bzw. Nilblaufluoreszenz. Dies ist möglich, da sich die Schalen auf Grund des Partikelaufbaus teilweise überlappen, obwohl in der mittleren Schale nur Fluorescein einpolymerisiert wurde.
- Ermittlung der Dicke von Schicht 2

### Äußere Schicht:

- Ermittlung des Quotienten 3 aus Aminocumarin- und Nilblau-Fluoreszenz
- Ermittlung der Dicke von Schicht 3

Nach diesen Regeln können die Mikropartikel des vorliegenden Beispiels dadurch unterschieden werden, daß Quotient 3 beider Partikelpopulationen sich messbar unterscheidet.
Quotient 3
Partikelpopulation 1: 0,5
Partikelpopulation 2: 1

Alle übrigen Quotienten und alle anderen Durchmesser und Schichtdicken der Partikel unterscheiden sich nicht.

Meßpunkt 1 wird bei der Auflösung 1 mit einem Objektiv mit 4-facher Vergrößerung aufgezeichnet. Die weiteren Messpunkte zur Bestimmung der Entwicklung der Ligandenfluoreszenz werden ebenfalls bei Auflösung 1 unter Verwendung eines Objektives mit 4-facher Vergrößerung aufgezeichnet. Zur korrekten Wiederfindung der Partikel werden die relativen Ortskoordinaten der Partikel aus Messpunkt 1 eingesetzt, um Ungenauigkeiten beim mehrfachen Anfahren der Messfelder ausgleichen zu können. Deshalb werden auch besonders bevorzugt als Singuletten immobilisierte Partikel in die Auswertung einbezogen, da diese leichter und sicherer identifiziert, dekodiert und wiedergefunden werden. Vorab quantifizierte Bleaching-Effekte der verwendeten Fluoreszenzfarbstoffe sind zu korrigieren, bevor aus den Daten die Ligandenfluoreszenzwerte abgeleitet werden. Die Zunahme als auch die Schnelligkeit der Zunahme der Ligandenfluoreszenz über verschiedene Messpunkte sind proportional zur Kinaseaktivität in der Probe (siehe auch Beispiel 2).

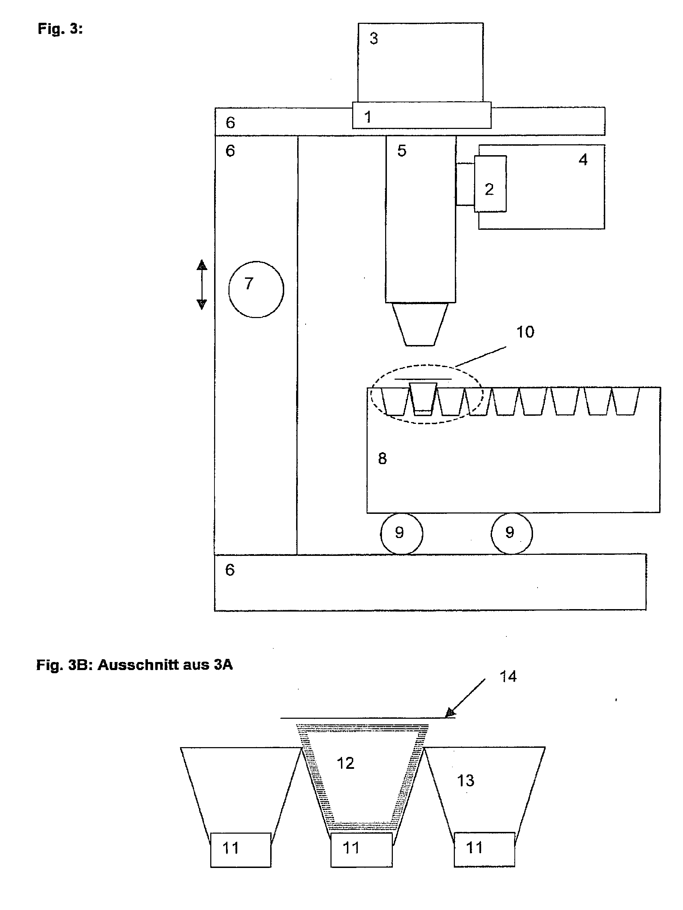

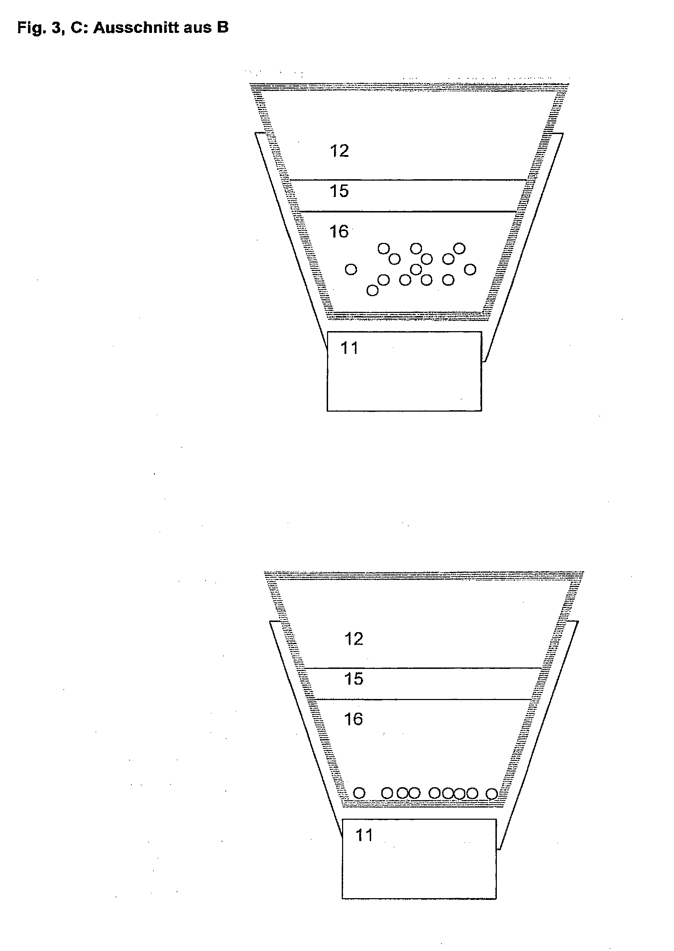

**Fig. 3****: Schematische Darstellung eines Gerätesystems (A) und einer Reaktionsumgebung (B) zur kombinierten Amplifikation von Targetsequenzen und Detektion der Amplifikate am Mikropartikelarray**
(A) Das vollautomatisch computergesteuerte Gerät stellt von seinen Leistungsparametern her eine Kombination aus eversen Fluoreszenzmikroskop und Thermocycler dar. An einem Stativ 6 mit motorisiertem z-Trieb befinden sich die optischen Komponenten mindestens bestehend aus einer Long-Distanz-Optik 5 für die Fluoreszenzdetektion mit einem 20X-Objektiv und Elementen zur Strahlführung und zum Bildaufbau ausgestattet. Die Anregung erfolgt über mindestens eine Xenonlampe oder mindestens zwei verschiedene LED 3 oder LASER 4 bevorzugt in den Spektralbereichen 350-400 nm; 450-500nm; 550-600 nm. Die konkreten Wellenlängen des Anregungslichtes richten sich nach den verwendeten Fluoreszenzfarbstoffen und werden bei Bedarf durch passende Filter 2 feinjustiert. Das von den Fluoreszenzfarbstoffen emittierte Licht wird durch entsprechende Emissionsfilter 1 vom Anregungslicht getrennt und durch eine sensitivierte Kamera aufgezeichnet 3. Der Objekttisch besitzt einen x/y-Antrieb der eine Genauigkeit von unter 0,1 mm aufweist und den Thermocycler mit Probenhalterung (hier für 96 Reaktionsgefäße mit 0,2 ml Fassungsvermögen) aufnimmt. Unter den Reaktionsgefäßen, die einen planaren Boden aufweisen, ist optional ein Magnet 11 (Dauermagnet oder ein Elektromagnet) positioniert, so dass paramagnetische Mikropartikel während des Messvorganges gut immobilisiert werden.
   Eine automatische Computersteuerung umfasst das PCR-Programm und damit das Temperaturprofil der PCR-Reaktion, die Filterwechsler, die Kamera, die Bildakquise und Bildverarbeitung, den x/y/z-Antrieb und die Steuerung des Anregungslichtes, sowie die Benutzeroberfläche, so dass während z.B. der PCR-Reaktion die Ausgabe der Amplifikat-Fluoreszenzen für die verschiedenen Partikelpopulationen gewährleistet werden kann. Alternativ zum eversen Fluoreszenzmikroskop kann das Gerät auch als inverses, Fluoreszenzmikroskop ausgeführt sein.
**(B)** Als Reaktionsgefäße werden z.B. NucleoSorb-8er-Riegel 12 verwendet, die mit einem Glasdeckglas verschlossen werden.
**(C)** Der PCR-Master-Mix wird zusammen mit der Proben-DNA 16 in das Reaktionsgefäß pipettiert und mit Öl überschichtet. Danach wird das Reaktionsgefäß optional mit einem Deckglas verschlossen und der PCR-/Messvorgang gestartet. Während die PCR-Zyklen ablaufen erfolgt die Messung der Fluoreszenzen bevorzugt in der Phase des Annealings (Anlagerung des Primers) bei isothermen Bedingungen. Alternativ erfolgt das Aufzeichnen einer Schmelzkurve am Ende der PCR. Die Mikropartikel sind während der PCR bevorzugt ständig immobilisiert durch kovalente Kopplung, Sedimentation oder magnetische Anziehung. Bei Verwendung von Elektromagneten können die Partikel während der PCR immobilisiert oder frei flottierend vorliegen und werden für jede Messung neu immobilisiert.
   1- Emissionsfilter
   2- Filter für Anregungslicht
   3- Kamera (CCD oder CMOS)
   4- LED oder Laser zur Anregung
   5- Long-Distanz-Optik
   6- Stativ
   7- z-Antrieb
   8- Thermocycler
   9- x/y-Antrieb
   10- Ausschnittsmarkierung
   11- Magnet
   12- NucleoSorb oder NucleoLink-8er-Riegel
   13- Reaktionsgefäßaufnahme im Thermoblock
   14- Deckel
   15- Überschichtung (z.B. Öl)
   16- Master-Mix + Proben-DNA

### Beispiele

### Beispiel 1: Nachweis des Erregers Herpes simplex Virus 1 in einer Probe

Ein Volumen von 5 µl aus Abstrichen von Lippenbläschen gereinigter Proben-DNS wird zu 25 µl Master-Mix folgender Zusammensetzung gegeben:
Primer 1 400 nM
Primer 2 300 nM
in Taq-DNS-Polymerase, Nukleotide, Magnesiumchlorid, Tris/HCl-Puffer und Tween 20 gelöst.

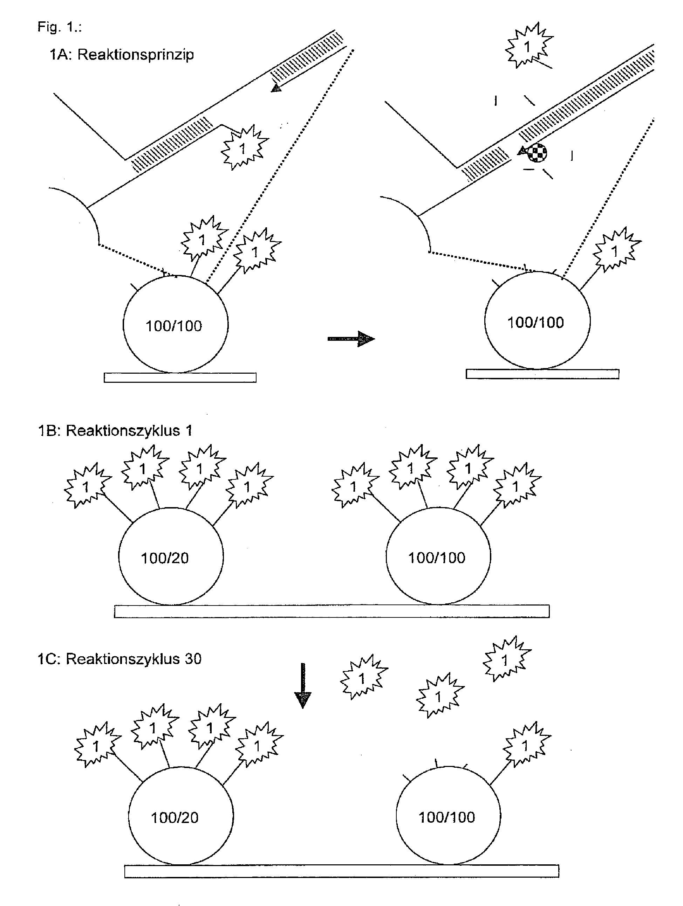

Der Reaktionsansatz wird in eine Kavität einer Nukleolink-Platte pipettiert und anschließend in den Thermocycler des Messgerätes (siehe Fig. 3) überführt und thermocyclisch amplifiziert. Das Reaktionsprinzip des Verfahrens ist schematisch in Fig. 1 dargestellt. Primer 1 und 2 binden an die Targetsequenz des *Herpes simplex-Virus 1-*Genoms sowie an den internen Standard. Durch eine DNS-Polymerase werden die von den Primern flankierten Fragmente unter geeigneten Bedingungen amplifiziert. Nach Denaturierung des Doppelstranges kann die Sonde auf der Partikeloberfläche, die eine Rhodamin-Markierung am 5'-Ende trägt, mit den komplementären Strängen des Amplifikates hybridiseren. Bei Verlängerung des Primers im nächsten Reaktionszyklus werden die hybridisierten Sonden durch die 5'-3'-Exonuklease der Taq-Polymerase degradiert und der Farbstoff freigeseetzt, was zu einer Abnahme der Rhodaminfluoreszenz bei Zunahme der Amplifkatmenge führt.

Die Partikel bestehen aus Polymetharcrylat und haben einen Durchmesser von 10 µm. Die Oberfläche der Partikel ist carboxymodifiziert und mit Hilfe einer Carbodiimid-Kopplung werden die 3'-aminomodifizierten Fangsonden und ein 3'-aminomodifiziertes und 5'-phosphoryliertes Poly-T(50)-Linkermolekül an die aktivierten Partikel entsprechend Standardprotokollen gekoppelt. Die mit Fangsonden und Linkermolekülen im Verhältnis 1:5 beschichteten Partikel werden dann ebenfalls über eine Carbodiimid-Kopplung an den Boden einer Nukleolink-Platte gebunden.

### Partikel 1:

Der Partikel enthält z.B. 100 Teile Aminocumarin und z.B. 20 Teile Nilblau (100/30).

### Partikel 2:

Der Partikel enthält z.B. 100 Teile Aminocumarin und z.B. 100 Teile Nilblau (100/100).

### Partikel 3:

Der Partikel enthält z.B. 100 Teile Aminocumarin und z.B. 0 Teile Nilblau (100/0).

Nachdem der Mastermix in die mit Partikeln beschichteten Reaktionsgefäße pipettiert und diese verschlossen wurden, werden die Partikelfluoreszenz für Aminocumarin und Nilblau unter Verwendung eines Objektivs mit 20-facher Vergrößerung aufgezeichnet. Die Partikel der Partikelpopulationen 1 und 2 werden lokalisiert und identifiziert. Die Rhodamin-Oberflächenfluoreszenz wird bevorzugt zwischen PCR-Zyklus 20-30 unter Verwendung eines Objektivs mit 4-facher Vergrößerung bei geringen Partikeldichten und mit 10-facher Vergrößerung bei hohen Partikeldichten aufgezeichnet solange bis Amplifikat eindeutig detektiert wurde, d.h. die Oberflächenfluoreszenz der *Herpes simplex Virus* 1-spezifischen Partikel (Partikel 1) und / oder von internen Standard-Partikeln (Partikel 2) deutlich im Vergleich zur Referenzfluoreszenz (Partikel 3) abgenommen hat. Die Referenzpartikel können optional genutzt werden, um etwaige unspezifische Effekte wie unerwünschte Nukleaseaktivitäten oder Bleaching-Effekte erkennen zu können.

### Sequenzen

Primer 1: 5'-CAT CAC CGA CCC GGA GAG GGA C-3' (SEQ ID No: 1)
Primer 2: 5'-GGG CCA GGC GCT TGT TGG TGT A-3' (SEQ ID No: 2)
Sonde *Herpes simplex-*Virus 1:
Rhodamin-5'-GGACTTTGTCCTCACCGCCGAACTGATTTTTTTTTTTTTTT-3'-Partikel 1 (SEQ ID No: 3)
Sonde Interner Standard: :
   Rhodamin-5'-GACCGCTTGCTGCAACTCTCTCAGTTTTTTTTTTTTTT-3'-Partikel 2 (SEQ ID No: 4)
Sonde für Referenzpartikel zum Ausschluß unspezischer Effekte:
   Rhodamin-5'-TTTTTTTTTTTTTTTTTTTTTTTT-3'-Partikel 3 (SEQ ID No: 5)

### Interner Standard:

Der Multiplex-Grad kann erhöht werden, indem weitere Zielsequenzen, wie z.B. Herpes simplex Virus 2, simultan im Reaktionsansatz amplifiziert und an weiteren Partikelpopulationen mit den jeweils spezifischen Fangsonden detektiert werden.

Es ist problemlos möglich dieses Testformat zur Charakterisierung von Enzymen wie z.B. Proteasen, Phosphatasen, Glycosidasen zu übertragen, die die Eigenschaft besitzen, die fluoreszenzmakierten Akzeptormoleküle auf der Oberfläche der Partikel zu degradieren.

### Beispiel 2: Kinaseassay zum Nachweis unterschiedlicher Substratspezifitäten

Ein Volumen von 5 µl einer ERK1-haltigen Probe wird zu 25 µl Reaktions-Mix folgender Zusammensetzung gegeben:

ATP, Magnesiumchlorid, EGTA, DTT, Tris/HCl-Puffer und Tween 20, gegebenenfalls Enzyminhibitoren, Rhodamin-markierter Anti-Phophopeptid-Antikörper gegebenenfalls unterschiedlicher Spezifität. Alternativ zum Anti-Phophopeptid-Antikörper können auch Phosphogruppenspezifische Fluoreszenzfarbstoffe eingesetzt werden. Im Falle anderer Modifikationen wie z.B. Glykosylierungen, können auch anderere Modifikationsspezifische Fluoreszenzfarbstoffe genutzt werden.

Zur Testung von Kinase-Inhibitoren werden in die Kavitäten einer Mikrotestplatte unterschiedlichen Reaktionsansätzen verschiedene Kinaseinhibitoren wie z.B. Staurosporin zugesetzt und die Reaktion an auf Partikel immobilisierten phosphorylierbaren Peptiden und Kontrollpeptiden aufgezeichnet. Bei Verwendung verschiedener phosphorylierbarer Peptide inkl. Kontrollpeptide ist es alternativ möglich möglich, unterschiedliche Substratspezifitäten im Multiplexansatz zu vergleichen.

Kann ein Peptid phosphoryliert werden, bindet simultan der Rhodamin-markierte Anti-phosphogruppen-Antikörper und erzeugt eine messbare Ligandenfluoreszenz.

Die Partikel bestehen aus Polymetharcrylat und haben einen Durchmesser von 10 µm. Die Oberfläche der Partikel ist carboxymodifiziert und mit Hilfe einer Carbodiimid-Kopplung werden die an die aktivierten Partikel entsprechend Standardprotokollen gekoppelt. Die mit Peptiden und gegebenenfalls mit Linkermolekülen im Verhältnis 1:5 beschichteten Partikel werden dann ebenfalls über eine Carbodiimid-Kopplung an den Boden einer Nukleolink-Platte gebunden. Alternativ können die Partikel durch Schwerkraft- oder Magnet-Sedimentation immobilisert werden.

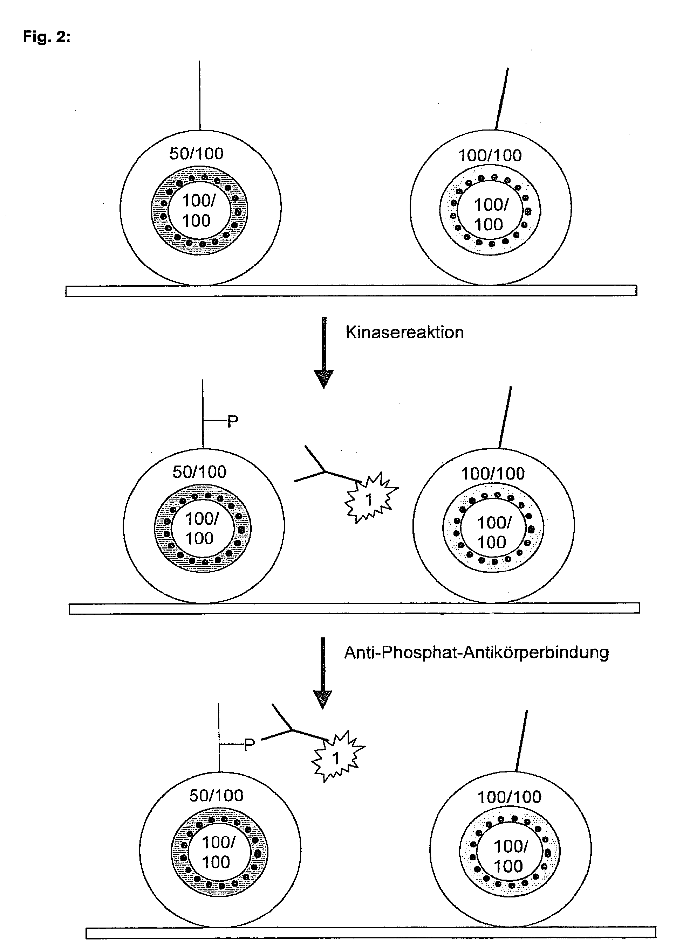

Folgende Partikel werden entsprechend Fig. 2 verwendet:

### Partikel 1:

Der Kern enthält z.B. 100 Teile Aminocumarin und z.B. 100 Teile Nilblau (100/100). Die mittlere Schicht enthält z.B. 100 Teile Fluorescein und die äußere Schicht enthält z.B. 50 Teile Aminocumarin und z.B, 100 Teile Nilblau.

### Partikel 2:

Der Kern enthält z.B. 100 Teile Aminocumarin und z.B. 100 Teile Nilblau (100/100). Die mittlere Schicht enthält z.B. 100 Teile Fluorescein und die äußere Schicht enthält z.B. 100 Teile Aminocumarin und z.B. 100 Teile Nilblau.

Partikel 1 trägt ein von ERK1 phosphorylierbares Peptid und Partikel 2 zu Referenzzwecken ein ähnliches aber nicht phosphorylierbares Peptid. Bei Verwendung weiterer Mikropartikelpopulationen können weitere phosphorylierbare Peptide in den Test integriert werden, was den Multiplex-Grad der analyse erhöht.

Nachdem die Partikelfluoreszenz für Aminocumarin und Nilblau unter Verwendung eines Objektivs mit 20-facher Vergrößerung aufgezeichnet und die Partikel der Partikelpopulationen 1 und 2 werden lokalisiert und identifiziert wurden, wird der Reaktions-Mix in die mit Partikeln beschichteten Reaktionsgefäße pipettiert und diese verschlossen. Die Entwicklung der Rhodamin-Ligandenfluoreszenz in Folge der Kinasereaktion wird in Abhängigkeit der Reaktionsgeschwindigkeit zu verschiedenen Zeitpunkten unter Verwendung eines Objektivs mit 4-facher Vergrößerung bei geringen Partikeldichten und mit 10-facher Vergrößerung bei hohen Partikeldichten aufgezeichnet solange bis ein eindeutiges Signal detektiert wurde.

Alternativ können nach dem hier dargelegten Prinzip viele andere Anwendungen zur Detektion von Antigenen und Antikörpern etabliert werden, mit aber auch ohne simultan ablaufender Enzymreaktion.

### Legende zu den Abbildungen

## Patentansprüche

1. Verfahren zur Multiplex-Analyse von mehreren Analyten umfassend die Schritte:
a) Einsatz eines Trägers, auf dem mindestens zwei verschiedene Mikropartikelpopulationen immobilisiert sind, wobei sich die verschiedenen Mikropartikelpopulationen in ihrer Fluoreszenzkodierung unterscheiden und mindestens zwei der unterschiedlich fluoreszenzkodierten Mikropartikelpopulationen jeweils Mikropartikel enthalten, die mit einer bestimmten, spezifischen Akzeptormolekülpopulation besetzt sind, wobei sich die Akzeptormolekülpopulationen der mindestens zwei unterschiedlichen fluoreszenzkodierten Mikropartikelpopulationen voneinander unterscheiden.
b) Messung der Fluoreszenz des Trägers aus Schritt a) mit einer optischen Auflösung 1 vor Inkontaktbringen des Trägers mit der zu analysierenden Probe oder vor dem Start der Reaktion,
wobei die Auflösung 1:
- eine Unterscheidung von Mikropartikel-Singletten, -Dubletten, - Tripletten, -Multipletten und -Monolayern erlaubt, und
- die Bestimmung der örtlichen Lage der einzelnen immobilisierten Mikropartikel der jeweiligen mindestens zwei unterschiedlichen Mikropartikelpopulationen auf dem Träger unter Berücksichtigung der jeweils unterschiedlichen Fluoreszenzkodierung der mindestens zwei verschiedenen Mikropartikelpopulationen gestattet.
c) Inkontaktbringen des Trägers aus Schritt a) mit der zu analysierenden Probe, wobei die Interaktion des jeweiligen Analyten mit dem für ihn spezifischen Akzeptormolekül auf dem entsprechenden immobilisierten Mikropartikel eine Fluoreszenzänderung hervorruft.
d) Vornahme mindestens einer weiteren Messung der Fluoreszenz des Trägers während oder nach dem Inkontaktbringen oder dem Start der Reaktion gemäß Schritt c) mit einer Auflösung 2.
e) Zuordnung der mit Auflösung 2 gemessenen Fluoreszenzwerte zu den gemäß Schritt b) örtlich auf den Träger identifizierten und einer bestimmten Akzeptormolekülpopulation zugeordneten, einzelnen Mikropartikel-Singletten, - Dubletten, -Tripletten, -Multipletten und -Monolayern.
f) Bestimmung der Fluoreszenzänderung durch Inkontaktbringen gemäß Schritt c) für jede örtlich identifizerte Mikropartikel-Singlette und jeden Mikropartikel in einer-Dublette, -Triplette, -Multiplette und -Monolayer auf dem Träger.

2. Verfahren nach Anspruch 1 **dadurch gekennzeichnet, dass** der strukturierte oder nicht strukturierte Träger gemäß Anspruch 1, Schritt a) mikroskopierbare, planare Bereiche aufweist.

3. Verfahren nach einem der Ansprüche 1 bis 2, **dadurch gekennzeichnet, dass** die Mikropartikel durch einen oder mehrere Fluoreszenzfarbstoffe im Partikel oder an der Partikeloberfläche fluoreszenzkodiert und zusätzlich über die Partikelgröße größenkodiert oder durch morphologische Muster strukturkodiert sind und somit distinkten Mikropartikelpopulationen zugeordnet werden können.

4. Verfahren nach einem der Ansprüche 1 bis 3, **dadurch gekennzeichnet, dass** die Mikropartikel aus einem Kern und mindestens einer Schale bestehen, wobei sich die Materialien von Kern und Schale in Zusammensetzung, Form, Dichte, Transparenz, Modifizierbarkeit unterscheiden können und unterschiedliche Fluoreszenzkodierungen mit mindestens einem Fluoreszenzfarbstoff aufweisen, wobei unterschiedliche oder identische Fluoreszenzfarbstoffe zur Anwendung kommen können.

5. Verfahren nach einem der Ansprüche 1 bis 4, **dadurch gekennzeichnet, dass** an den Träger gemäß Anspruch 1, Schritt a) zusätzlich lebende oder abgetötete Zellen oder Zellen in Kombination mit Mikropartikeln immobilisiert sind.

6. Verfahren nach einem der Ansprüche 1 bis 5, **dadurch gekennzeichnet, dass** der Analyt direkt oder indirekt über ein weiteres Molekül mit einer Ligandenfluoreszenz oder einem Quencher markiert wird.

7. Verfahren nach einem der Ansprüche 1 bis 6, **dadurch gekennzeichnet, dass** die Verringerung der Fluoreszenz durch Quenching der Oberflächenfluoreszenz oder Ablösung der Oberflächenfluoreszenz von der Partikeloberfläche während der Degradation der Akzeptormoleküle während der Reaktion eintritt.

8. Verfahren nach einem der Ansprüche 1 bis 6, **dadurch gekennzeichnet, dass** die Zunahme der Ligandenfluoreszenz durch Aufhebung des Quenching von Reportersystemen, durch FRET oder durch lokale Anreicherung von Ligandenfluoreszenzmolekülen auf der Partikeloberfläche durch Interaktion mit den Akzeptormolekülen oder dem Analyten sowie durch Verdrängung von Quenchermolekülen während der Reaktion eintritt.

9. Verfahren nach Anspruch 8, **dadurch gekennzeichnet**, das dem Reaktionsgemisch zur Kontraststeigerung der Ligandenfluoreszenz ein Quencherfarbstoff oder lichtabsorbierende Nanopartikel zugesetzt werden.

10. Verfahren nach einem der Ansprüche 1 bis 9, **dadurch gekennzeichnet, dass** ein Messgerät zur Messung von Fluoreszenzänderungen eingesetzt wird, welches:
- eine vollautomatische Steuerung mittels Computer,
- einen Thermocycler für die schnelle Temperierung der Proben, mit einer Heiz-/Kühlrate von 3-20 °C pro Sekunde, welcher einen Reaktionsbereich mit mehreren temperierbaren Aufnahmen für Träger gemäß Anspruch 1, Schritt a) aufweist,
- eine Positioniereinrichtung für den Thermocycler und/oder die Reaktionsumgebung die thermozyklisch steuerbar ist,
- eine oder mehrere dem Reaktionsbereich zugeordnete Beleuchtungseinrichtung(en) mit denen Anregungslicht strahlbar ist,
- eine Optikeinrichtung die vorzugsweise für die Fluoreszenzdetektion im Durchlicht bzw. Auflicht mit entsprechenden optischen Filtern geeignet ist,
- eine oder mehrere Detektoreinrichtung(en) (z.B. CCD, CMOS), die in Abhängigkeit von einer gemessenen Fluoreszenzintensität Bilder erzeugt, und
- eine Auswerteeinheit, die aus den Bildern Messwerte erzeugt,
umfaßt.

11. Verfahren nach einem der Ansprüche 1 bis 10, **dadurch gekennzeichnet, daß** während der Aufzeichnung weiterer Messpunkte nur die Oberflächen- bzw. Ligandenfluoreszenz einer durch Ortskoordinaten definierten Pixelanazahl der nach Messpunkt 1 vorselektierten Partikel aufgezeichnet bzw. weiterverarbeitet wird.

12. Verfahren nach einem der Ansprüche 1 bis 11, **dadurch gekennzeichnet, dass** die verschiedenen Partikelfluoreszenzen zueinander und / oder mit der Oberflächenfluoreszenz bzw. mit der Partikelgröße ins Verhältnis gesetzt werden, um die mindestens zwei unterschiedlichen Mikropartikelpopulationen zu dekodieren.

13. Verfahren nach einem der Ansprüche 1 bis 12, **dadurch gekennzeichnet, dass** die im Zeitverlauf öder in unterschiedlichen Reaktionszyklen aufgezeichneten Messwerte der weiteren Messpunkte einer Messreihe ein und des selben Messortes auf einen oder mehrere Messwerte derselben Messreihe, desselben Messortes oder auch anderer Messorte referenziert werden.

14. Verfahren nach einem der Ansprüche 1 bis 13, **dadurch gekennzeichnet, dass** die verschiedenen Partikelfluoreszenzen verschiedener Partikelschichten zueinander und / oder mit der Oberflächen-/Ligandenfluoreszenz bzw. mit der Partikelgröße ins Verhältnis gesetzt werden, um die Mikropartikelpopulationen zu dekodieren und/oder die Oberflächen- bzw. die Ligandenfluoreszenz zu referenzieren.

## Claims

1. A method for the multiplex analysis of multiple analytes comprising the steps:
a) Use of a support on which at least two different microparticle populations are immobilized, wherein the different microparticle populations differ in their fluorescence coding and at least two of the differently fluorescence-coded microparticle populations contain microparticles respectively, which are occupied by a particular, specific acceptor molecule population, wherein the acceptor molecule populations of the at least two differently fluorescence-coded microparticle populations differ from one another.
b) Measurement of the fluorescence of the support from step a) using an optical resolution 1 prior to bringing the support into contact with the sample to be analyzed or prior to the start of the reaction,
said resolution 1:
- permitting the differentiation of microparticle singlets, doublets, triplets, multiplets and monolayers, and
- allowing the determination of the localized position of the individual immobilized microparticles of the at least two different microparticle populations respectively on the support, taking account of the different fluorescence coding respectively of the at least two different microparticle populations.
c) Bringing the support from step a) into contact with the sample to be analyzed, wherein the interaction of the respective analyte with the specific acceptor molecule for it produces a modification in fluorescence on the relevant immobilized microparticle.
d) Taking of at least one additional measurement of the fluorescence of the support during or after the contact made or the start of the reaction in step c) with a resolution 2.
e) Assignment of the fluorescence values measured with resolution 2 for the individual microparticle singlets, doublets, triplets, multiplets and monolayers which have been locally identified on the support in step b) and assigned to a particular acceptor molecule population.
f) Determination of the modification in fluorescence by the contact made in step c) for each locally identified microparticle singlet and each microparticle in a doublet, triplet, multiplet and monolayer on the support.

2. The method according to Claim 1, **characterized in that** the structured or non-structured support according to step a) of Claim 1 has microscopable, planar areas.

3. The method according to any one of Claims 1 to 2, **characterized in that** the microparticles are fluorescence coded by one or more fluorescent dyes in the particle or on the particle surface and, in addition, are size coded by means of the particle size or are structure coded by morphological patterns and can therefore be assigned to distinct microparticle populations.

4. The method according to any one of Claims 1 to 3, **characterized in that** the microparticles consist of a nucleus and at least one shell, wherein the materials of the nucleus and shell can differ in composition, form, density, transparency, modifiability and have different fluorescence codings with at least one fluorescent dye, wherein different or identical fluorescent dyes can be used.

5. The method according to any one of Claims 1 to 4, **characterized in that** living or killed cells or cells in combination with microparticles are, in addition, immobilized on the support according to step a) of Claim 1.

6. The method according to any one of Claims 1 to 5, **characterized in that** the analyte is marked directly or indirectly by means of a further molecule with ligandfluorescence or a quencher.

7. The method according to any one of Claims 1 to 6, **characterized in that** the reduction of the fluorescence by means of quenching of the surface fluorescence or the removal of the surface fluorescence from the particle surface occurs during the degradation of the acceptor molecules during the reaction.

8. The method according to any one of Claims 1 to 6, **characterized in that** the increase of the ligandfluorescence by cancellation of the quenching by reporter systems, by FRET or by local enrichment of ligandfluorescent molecules on the particle surface occurs as a result of interaction with the acceptor molecules or the analyte as well as by means of suppression of quencher molecules during the reaction.

9. The method according to Claim 8, **characterized in that** a quencher dye or light-absorbing nanoparticles is/are added to the reaction mixture in order to increase the contrast of the ligandfluorescence.

10. The method according to any one of Claims 1 to 9, **characterized in that** a measuring device for measuring modifications in fluorescence is used, which comprises:
- fully automatic control by means of computer,
- a thermocycler for the rapid tempering of the samples, with a heating/cooling rate of 3-20°C per second, which has a reaction zone with several temperable receptacles for the support according to step a) of Claim 1,
- a positioning device for the thermocycler and/or the reaction environment which can be thermocyclically controlled,
- one or more lighting unit(s) which is/are assigned to the reaction zone, which can radiate excitation light,
- an optical device which is preferably suitable for detecting fluorescence in the transmitted light and/or incident light with appropriate optical filters,
- one or more detector device(s) (e.g. CCD, CMOS), which produce(s) images as a function of a measured fluorescence intensity, and
- an evaluation unit which produces measuring values from the images.

11. The method according to any one of Claims 1 to 10, **characterized in that** during the recording of further measuring points only the surface fluorescence and/or ligandfluorescence of a numbers of pixels of the preselected particles according to measuring point 1, which are defined by location coordinates, is/are recorded and/or further processed.

12. The method according to any one of Claims 1 to 11, **characterized in that** the different particle fluorescences are compared with one another and/or with the surface fluorescence and/or with the particle size, in order to decode at least two different microparticle populations.

13. The method according to any one of Claims 1 to 12, **characterized in that** the measuring values of the additional measuring points of a measuring series of one and the same measuring location, which are recorded over the course of time or in different reaction cycles, are referenced to one or more measuring values of the same measuring series, the same measuring location or also of other measuring locations.

14. The method according to any one of Claims 1 to 13, **characterized in that** the different particle fluorescences of different particle layers are compared with one another and/or with the surface fluorescence/ligandfluorescence and/or with the particle size, in order to decode the microparticle populations and/or the surface fluorescence and/or the ligandfluorescence.

## Revendications

1. Procédé d'analyse multiplex de plusieurs analytes, comprenant les étapes suivantes :
a) Mise en oeuvre d'un support, sur lequel au moins deux populations de microparticules différentes sont immobilisées, les populations de microparticules différentes se distinguant par leur codage par fluorescence, et au moins deux des populations de microparticules codées de façon différente par leur fluorescence contenant respectivement des microparticules qui sont occupées par une population de molécules acceptrices spécifique définie, les populations de molécules acceptrices des populations de microparticules au moins au nombre de deux codées de façon différente par leur fluorescence se distinguant les unes des autres.
b) Mesure de la fluorescence du support issu de l'étape a) avec une résolution optique 1 avant la mise en contact du support avec l'échantillon à analyser ou avant le démarrage de la réaction,
la résolution 1 :
- permettant de distinguer entre des singlettes, doublettes, triplettes, multiplettes et monocouches de microparticules, et
- autorisant la détermination de la position locale des différentes microparticules immobilisées des différentes populations respectives de microparticules différentes au moins au nombre de deux sur le support en prenant en compte le codage par fluorescence respectivement différent des populations de microparticules différentes au moins au nombre de deux.
c) Mise en contact du support issu de l'étape a) avec l'échantillon à analyser, l'interaction de l'analyte respective avec la molécule acceptrice qui lui est spécifique provoquant une variation de la fluorescence sur la microparticule immobilisée correspondante.
d) Réalisation d'au moins une autre mesure de la fluorescence du support pendant ou après la mise en contact ou le démarrage de la réaction selon l'étape c) avec une résolution 2.
e) Affectation des valeurs de fluorescence mesurées avec la résolution 2 aux différentes singlettes, doublettes, triplettes, multiplettes et monocouches de microparticules identifiées selon l'étape b) localement sur le support et affectées à une population de molécules acceptrices déterminée.
f) Détermination de la variation de la fluorescence par mise en contact selon l'étape c) pour chaque singlette de microparticules identifiée localement et chaque microparticule dans une doublette, triplette, multiplette et monocouche de microparticules sur le support.

2. Procédé selon la revendication 1, **caractérisé en ce que** le support structuré ou non structuré selon la revendication 1, étape a), présente des zones planaires observables au microscope.

3. Procédé selon une des revendications 1 à 2, **caractérisé en ce que** les microparticules peuvent être codées par fluorescence par un ou plusieurs colorants fluorescents dans la particule et en plus codées par taille par le biais de la taille de particule ou codées par structure par des modèles morphologiques et peuvent ainsi être affectées à des populations de microparticules distinctes.

4. Procédé selon une des revendications 1 à 3, **caractérisé en ce que** les microparticules se composent d'un noyau et d'au moins une enveloppe, les matériaux du noyau et de l'enveloppe pouvant se distinguer par leur composition, leur forme, leur densité, leur transparence, leur possibilité de modification et présentent des codages par fluorescence différents avec au moins un colorant fluorescent, des colorants fluorescents différents ou identiques pouvant être utilisés.

5. Procédé selon une des revendications 1 à 4, **caractérisé en ce que**, en plus, des cellules vivantes ou tuées ou des cellules combinées avec des microparticules sont immobilisées sur le support selon la revendication 1, étape a).

6. Procédé selon une des revendications 1 à 5, **caractérisé en ce que** l'analyte est marquée, directement ou indirectement par le biais d'une autre molécule, avec une fluorescence de ligand ou un quencher.

7. Procédé selon une des revendications 1 à 6, **caractérisé en ce que** la réduction de la fluorescence par quenching de la fluorescence de surface ou par détachement de la fluorescence de surface à partir de la surface de particule intervient pendant la dégradation des molécules acceptrices pendant la réaction.

8. Procédé selon une des revendications 1 à 6, **caractérisé en ce que** l'augmentation de la fluorescence de ligand est suscitée par la suppression du quenching de systèmes rapporteurs, par un FRET ou par l'enrichissement local de molécules de fluorescence de ligand à la surface de particule par interaction avec les molécules acceptrices ou avec l'analyte ainsi que par le refoulement de molécules de quencher pendant la réaction.

9. Procédé selon la revendication 8, **caractérisé en ce qu'**un colorant quencher ou des nanoparticules absorbant la lumière est/sont ajouté(s) au mélange de réaction pour augmenter le contraste de la fluorescence de ligand.

10. Procédé selon une des revendications 1 à 9, **caractérisé en ce que**, pour la mesure de variations de fluorescence, il est mis en oeuvre un appareil de mesure qui comprend :
- une commande entièrement automatique au moyen d'ordinateurs,
- un thermocycleur pour un équilibrage rapide de température des échantillons, à un rythme chauffage/refroidissement de 3-20 °C par seconde, qui présente une plage de réaction, avec plusieurs logements pour support pouvant être équilibrés en température selon la revendication 1, étape a),
- un dispositif de positionnement pour le thermocycleur et/ou l'environnement de réaction qui peut être commandé de façon thermocyclée,
- un ou plusieurs dispositif(s) d'éclairage affectés à la zone de réaction, avec lequel/lesquels la lumière d'excitation peut être projetée,
- un dispositif optique qui est de préférence approprié pour la détection de fluorescence dans la lumière de transmission ou respectivement la lumière incidente avec des filtres optiques correspondants,
- un ou plusieurs dispositif(s) de détection (par ex. CCD, CMOS) qui produit/produisent des images en fonction d'une intensité de fluorescence mesurée, et
- une unité d'analyse qui produit des valeurs de mesure à partir des images.

11. Procédé selon une des revendications 1 à 10, **caractérisé en ce que**, pendant l'enregistrement de points de mesure supplémentaires, seule la fluorescence de surface ou respectivement la fluorescence de ligand d'un nombre de pixels, défini par des coordonnées de localisation, des particules présélectionnées selon le point de mesure 1 est enregistrée ou soumise à un traitement complémentaire.

12. Procédé selon une des revendications 1 à 11, **caractérisé en ce que** les différentes fluorescences des particules sont mises en rapport les unes avec les autres et/ou avec la fluorescence de surface ou respectivement avec la taille de particules afin de décoder les différentes populations de microparticules au moins au nombre de deux.

13. Procédé selon une des revendications 1 à 12, **caractérisé en ce que** les valeurs de mesure, enregistrées au fil du temps ou dans des cycles de réaction différents, des points de mesure supplémentaires d'une série de mesure du même lieu de mesure sont référencées sur une ou plusieurs valeurs de mesure de la même série de mesure, du même lieu de mesure ou également d'autres lieux de mesure.

14. Procédé selon une des revendications 1 à 13, **caractérisé en ce que** les différentes fluorescences des particules de différentes couches de particules sont mises en rapport les unes avec les autres et/ou avec la fluorescence de surface/fluorescence de ligand ou respectivement avec la taille de particules afin de décoder les populations de microparticules et/ou pour référencer la fluorescence de surface ou respectivement la fluorescence de ligand.


### SEQUENCE LISTING

<110> Attomol GmbH
<120> verfahren zur Durchführung und Auswertung Mix & Measure Assays für die Messung von Reaktionskinetiken sowie von Konzentrationen und Affinitäten von Analyten im Multiplexformat
<130> P471207 PCT2
<150> DE 10 2007 031 137.2-41
   <151> 2007-06-29
<160> 6
<170> PatentIn version 3.3
<210> 1
   <211> 22
   <212> DNA
   <213> artificial sequence
<220>
   <223> primer
<400> 1
   catcaccgac ccggagaggg ac 22
<210> 2
   <211> 22
   <212> DNA
   <213> artificial sequence
<220>
   <223> primer
<400> 2
   gggccaggcg cttgttggtg ta 22
<210> 3
   <211> 41
   <212> DNA
   <213> artificial sequence
<220>
   <223> sonde
<400> 3
   ggactttgtc ctcaccgccg aactgatttt tttttttttt t 41
<210> 4
   <211> 38
   <212> DNA
   <213> artificial sequence
<220>
   <223> sonde
<400> 4
   gaccgcttgc tgcaactctc tcagtttttt tttttttt 38
<210> 5
   <211> 24
   <212> DNA
   <213> artificial sequence
<220>
   <223> sonde
<400> 5
   tttttttttt tttttttttt tttt 24
<210> 6
   <211> 160
   <212> DNA
   <213> artificial sequence
<220>
   <223> internal standard
<400> 6
   catcaccgac ccggagaggg acccagcgtg gaccgcttgc tgcaactctc tcagggccag    60
   gcggtgaagg gcaatcagct gttgcccgtc tcgctggtga aaagaaaaac caccctggcg   120
   cccaatacgc aaaccgccta caccaacaag cgcctggccc                         160